# EUROPEAN PATENT APPLICATION

(11) **EP 4 778 993 A1**
(43) Date of publication of application: **22.07.2026**
(21) Application number: 24865316.4
(22) Date of filing: 03.09.2024
(51) Int. Cl.: C09K 9/02, C07D 311/94, C08L 33/06, C08L 101/00, G02C 7/10

(54) **PHOTOCHROMIC COMPOSITION, CURABLE COMPOSITION, OPTICAL ARTICLE, LENS, AND EYEGLASSES**

(30) Priority: 11.09.2023 JP 2023147192
(71) Applicant: Tokuyama Corporation, Shunan-shi, Yamaguchi 745-8648 (JP)
(72) Inventor: MIYAZAKI, Masayuki, Shunan-shi, Yamaguchi 745-8648 (JP)
(74) Representative: Kraus & Lederer PartGmbB
(86) International application number: PCT/JP2024/031604
(87) International publication number: WO 2025/057814

(57) **Abstract**

Provided is a photochromic composition containing: a first photochromic compound represented by formula (1); and a second photochromic compound. The second photochromic compound includes at least one selected from the group consisting of: second A photochromic compounds that are represented by formula (1) and that have structures different from that of the first photochromic compound; second B photochromic compounds represented by formula (2); and second C photochromic compounds represented by formula (3). Also provided are a curable composition, an optical article, a lens, and eyeglasses, which all contain the photochromic composition.

## Description

### TECHNICAL FIELD

The present invention relates to a photochromic composition, a curable composition, an optical article, a lens, and eyeglasses.

### BACKGROUND ART

Photochromic compounds are compounds that can reversibly take two isomeric forms with different absorption spectra upon irradiation with light including ultraviolet light such as sunlight or light from a mercury vapor lamp. In general, a compound in a colorless or decolored state quickly changes in color and is isomerized (chromogenic reaction) to a colored or color-developed state by irradiation with ultraviolet light. For example, the photochromic compounds have been studied and developed as materials for photochromic lenses.

In applications of such photochromic lenses, the photochromic compounds may require the following properties:
(I) a degree of coloration in a visible light range before irradiation with ultraviolet light (hereinafter referred to as "initial coloration") is low;
(II) a color developing density quickly reaches a saturation level from the beginning of irradiation with ultraviolet light (hereinafter also referred to as "high color developing sensitivity");
(III) a color quickly returns to its original state after irradiation with ultraviolet light is stopped (hereinafter referred to as "color fading rate"); and
(IV) repetition durability of the above-mentioned reversible action is high.

A number of chromene compounds have been studied as photochromic compounds that satisfy these properties. For example, a chromene compound represented by Formula (A) below (see Patent Document 1), a chromene compound represented by Formula (B) below (see Patent Document 2), and a chromene compound represented by Formula (C) below (see Patent Document 3) have been known. Many photochromic compositions in which a plurality of photochromic compounds are mixed to achieve the desired photochromic property have also been investigated (see Patent Documents 4, 5, and 6).

A photochromic lens into which such a photochromic compound is incorporated has been manufactured in various ways. For example, it is manufactured by a method for impregnating a surface of the lens with the photochromic compound. A cured product of a photochromic curable composition may be used as the photochromic lens. Furthermore, there is also a method for obtaining a photochromic laminate by laminating a photochromic layer, which is a cured product of a photochromic curable composition, on an optical substrate. Among these, the method for laminating a photochromic layer on an optical substrate is beneficial because it can be applied to a variety of optical substrates. A photochromic curable composition includes, for example, a matrix material such as (meth)acrylate or isocyanate and a photochromic compound (see Patent Documents 7, 8, and 9).

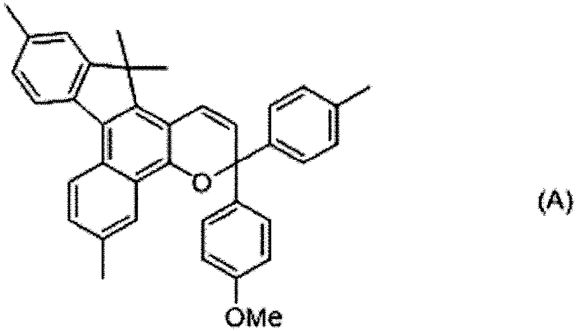

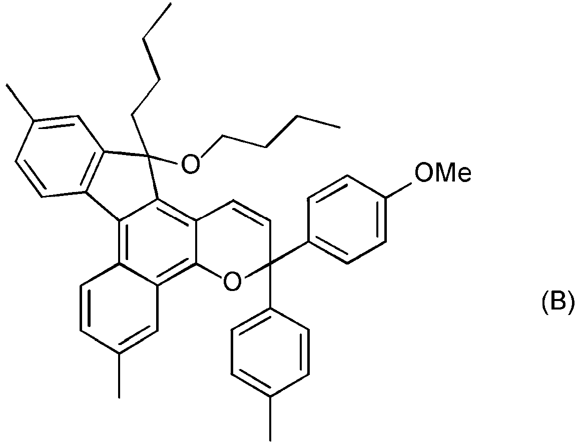

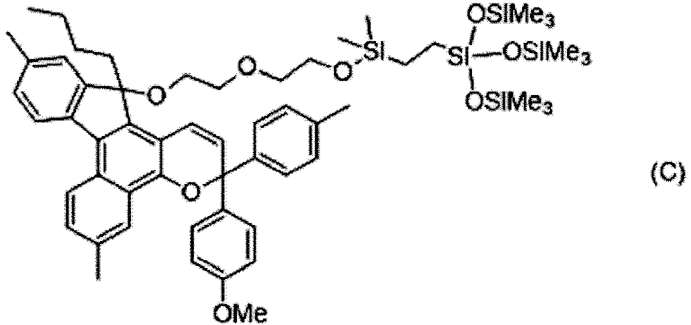

### Citation List

### Patent Documents

Patent Document 1: PCT International Publication No. WO1996/014596
Patent Document 2: PCT International Publication No. WO2004/085568
Patent Document 3: PCT International Publication No. WO2011/053615
Patent Document 4: PCT International Publication No. WO2022/138967
Patent Document 5: PCT International Publication No. WO2022/138965
Patent Document 6: Japanese Unexamined Patent Application, Publication No. 2023-037697
Patent Document 7: PCT International Publication No. WO2011/125956
Patent Document 8: PCT International Publication No. WO2020/094772
Patent Document 9: Japanese Unexamined Patent Application, Publication No. 2021-59681
Patent Document 10: PCT International Publication No. WO2018/235771
Patent Document 11: PCT International Publication No. WO2013/042800
Patent Document 12: Japanese Unexamined Patent Application, Publication No. 2018-62496
Patent Document 13: U.S. Patent Application, Publication No. 2006/0022176

### DISCLOSURE OF THE INVENTION

### Problems to be Solved by the Invention

An object of the present invention is to provide a photochromic composition with excellent solubility and durability; and a curable composition, an optical article, a lens, and eyeglasses including the photochromic composition.

### Means for Solving the Problems

According to the present disclosure, a photochromic composition is provided. The photochromic composition includes a first photochromic compound represented by Formula (1) below and a second photochromic compound. The second photochromic compound includes at least one selected from the group consisting of a second photochromic compound A represented by Formula (1) below and having a different structure from the first photochromic compound, a second photochromic compound B represented by Formula (2) below, and a second photochromic compound C represented by Formula (3) below.

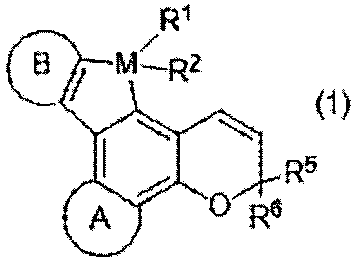

In Formula (1) above, M is C, Si, or Ge.

Ring A and Ring B are each independently a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings.

R¹ and R² are each independently a substituted or unsubstituted alkyl group or a group represented by Formula (2a) below, and have different structures from each other.

-Q¹-(X¹Q²)a-X²Q³ (2a)

In Formula (2a) above, Q¹ is an alkylene group or a haloalkylene group. Q² is an alkylene group or a haloalkylene group. Q³ is an alkyl group or a haloalkyl group. X¹ and X² are each independently O, S, NR⁷⁰⁰, PR⁷⁰¹, or P(=O). R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group. a is an integer of 0 or more and 10 or less.

R⁵ and R⁶ are each independently a hydrogen atom or a substituent.

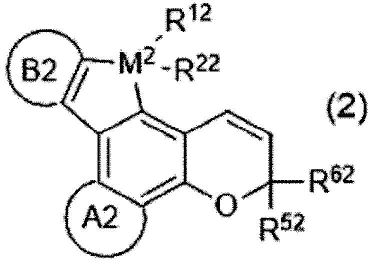

In Formula (2) above, M² is C, Si, or Ge.

Ring A2 and Ring B2 are each independently a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings.

R¹² and R²² are each a substituted or unsubstituted alkyl group or a group represented by Formula (2a) above and have the same structure as each other.

R⁵² and R⁶² are each independently a hydrogen atom or a substituent.

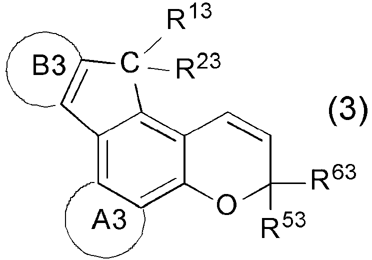

In Formula (3) above, Ring A3 and Ring B3 are a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings.

R¹³ and R²³ are taken together with a carbon atom to which they are attached to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the above-mentioned aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the above-mentioned heterocyclic ring.

R⁵³ and R⁶³ are each independently a hydrogen atom or a substituent.

According to the present disclosure, a curable composition is provided. The curable composition includes a photochromic composition of the present disclosure; and at least one selected from the group consisting of a radical polymerizable monomer, a cationic polymerizable monomer, a compound having a polymerization reactive group, and a (thio)urethane(urea) polymer.

According to the present disclosure, an optical article is provided. The optical article includes a cured product of a curable composition of the present disclosure.

According to the present disclosure, a lens is provided. The lens includes a photochromic compound of the present disclosure.

According to the present disclosure, eyeglasses are provided. The eyeglasses include the lens of the present disclosure.

### Effects of the Invention

The present invention provides a photochromic composition with excellent solubility and durability; and a curable composition, an optical article, a lens, and eyeglasses including the photochromic composition.

### PREFERRED MODE FOR CARRYING OUT THE INVENTION

A photochromic layer in a photochromic laminate may be required to be relatively thin in order to take advantage of a property of an optical substrate. However, a thinner photochromic layer may result in a decrease in repetition durability. Furthermore, a photochromic compound needs to be dispersed at a high concentration in the photochromic layer to exert a sufficient photochromic property in a thin film. Therefore, in addition to the above requirements, the photochromic compound may also be required to have high solubility in a matrix material and high dispersibility in a matrix. When the photochromic compound is dissolved in the matrix material at a high concentration, a mixture of the photochromic compound and the matrix material is sometimes heated to dissolve them. However, even if they are dissolved by heating, the thus-dissolved photochromic compound may recrystallize and precipitate after cooling. Therefore, the photochromic compound may also be required to have long-term solubility so that the photochromic compound does not precipitate from dissolution in the matrix material until formation of a photochromic laminate. A photochromic compound with extremely low crystallinity, such as a photochromic compound that is liquid at room temperature, is highly soluble in a matrix material. However, such a photochromic compound is less likely to crystallize, and thus, a trace impurity is difficult to remove through purification. If a photochromic compound including such an impurity is used, the impurity may degrade the photochromic compound in the photochromic layer, making it difficult for the photochromic compound to undergo an isomerization reaction. In other words, durability may be reduced.

The photochromic composition of the present disclosure includes a first photochromic compound having a backbone represented by Formula (1) below and a second photochromic compound. The second photochromic compound includes at least one selected from the group consisting of a second photochromic compound A represented by Formula (1) below and having a different structure from the first photochromic compound, a second photochromic compound B represented by Formula (2) below, and a second photochromic compound C represented by Formula (3) below.

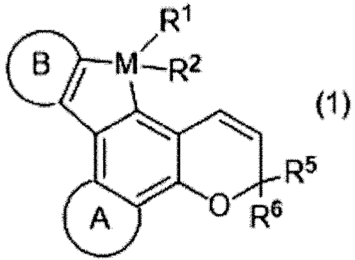

In Formula (1) above, M is C, Si, or Ge. Ring A and Ring B are each independently a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. R¹ and R² are each independently a substituted or unsubstituted alkyl group or a group represented by Formula (2a) and have different structures from each other. R⁵ and R⁶ are each independently a hydrogen atom or a substituent.

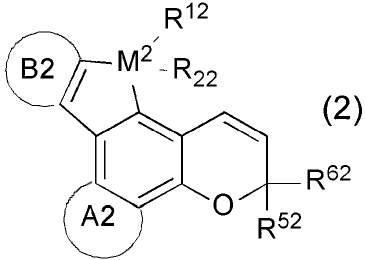

In Formula (2) above, M² is C, Si, or Ge. Ring A2 and Ring B2 are each independently a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. R¹² and R²² are each a substituted or unsubstituted alkyl group or a group represented by Formula (2a) and have the same structure as each other. R⁵² and R⁶² are each independently a hydrogen atom or a substituent.

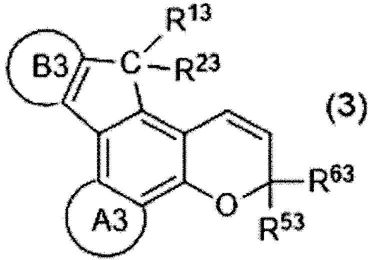

In Formula (3) above, Ring A3 and Ring B3 are each independently a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. R¹³ and R²³ are taken together with a carbon atom to which they are attached to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the above-mentioned aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the above-mentioned heterocyclic ring. R⁵³ and R⁶³ are each independently a hydrogen atom or a substituent.

This photochromic composition has excellent durability and solubility. The reason for this is unclear, but it is believed to be as follows by the present inventors.

The photochromic compound of Formula (1) above is characterized by attachment of R¹ and R², which are alkyl groups having different structures from each other, to an atom M at position 13. Solubility of the photochromic compound in a matrix material is believed to be affected by both its affinity for the matrix material and flexibility of a crystal structure of the photochromic compound. The photochromic compound of Formula (1) is substituted with alkyl groups having different structures from each other at an atom M at position 13, which is believed to moderately reduce molecular symmetry and weaken an intermolecular interaction of the photochromic compound. As a result, flexibility of a crystal structure of the photochromic compound may be increased. However, since crystallinity is not completely lost, a photochromic compound with high purity can be obtained by, for example, recrystallization. Therefore, it is believed that a decrease in durability due to degradation of the photochromic compound caused by an impurity can be suppressed.

Additionally, even after the photochromic compound is dissolved into the matrix material, a growth rate of a crystal is reduced due to low molecular symmetry, making it less likely to crystallize.

A photochromic composition of the present disclosure is used as a mixture of a first photochromic compound of Formula (1) above and a second photochromic compound having a different structure from the first photochromic compound. This is believed to further reduce the growth rate of the crystal when dissolved in the matrix material compared to a case of the first photochromic compound alone, and thus provides superior long-term solubility. Such an effect is believed to similarly inhibit growth of the crystal even when mixed with a second photochromic compound such as of Formula (2) above or Formula (3) above which has relatively high molecular symmetry and crystallinity, and is expected to increase the long-term solubility, which is a property of continuing to dissolve over a long period of time, compared to when only the second photochromic compound such as of Formula (2) above or Formula (3) above is included.

Thus, the photochromic composition of the present disclosure can exhibit high durability while achieving high solubility in various matrix materials.

In light of the above, the photochromic composition of the present disclosure can be used to provide a photochromic curable composition with excellent solubility in a curable composition and thus achieve a cured product or an optical article with excellent durability.

A photochromic composition of the present disclosure will be described in detail.

### <<Photochromic composition>>

A photochromic composition of the present disclosure includes a first photochromic compound and a second photochromic compound, and may be a mixture of the first photochromic compound and the second photochromic compound.

### <First photochromic compound>

A first photochromic compound is represented by Formula (1) below.

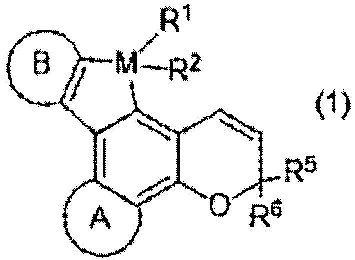

### (M)

In Formula (1) above, M is C, Si, or Ge. M is preferably C.

### (Ring A and Ring B)

In Formula (1) above, Ring A and Ring B are each a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. A substituent that Ring A or Ring B has may be the same as R³ and R⁴ described below.

Examples of the aromatic hydrocarbon ring include a benzene ring, a cyclotetradecaheptaene ring, etc.

Examples of the aromatic heterocyclic ring include a furan ring, a thiophene ring, a pyridine ring, etc.

Examples of the fused polycyclic ring include a naphthalene ring, a fluorene ring, an anthracene ring, a phenanthrene ring, a tetracene ring, a pentacene ring, a benzopyrene ring, a chrysene ring, a pyrene ring, a triphenylene ring, a perylene ring, a benzofuran ring, a benzothiophene ring, a quinoline ring, an isoquinoline ring, an indole ring, a pyrimidine ring, a quinazoline ring, a pyridazine ring, a cinnoline ring, a phthalazine ring, a 1,2,3-, 1,2,4-, or 1,3,5-triazine ring, a carbazole ring, a benzoxazole ring, an isothiazole ring, etc.

Among these, a benzene ring, a naphthalene ring, a fluorene ring, a phenanthrene ring, a pyrene ring, a furan ring, a thiophene ring, or a pyridine ring is preferred, and a benzene ring, a naphthalene ring, a fluorene ring, or a phenanthrene ring is further preferred.

### (R¹ and R²)

In Formula (1) above, R¹ and R² are each independently a linear or branched alkyl group or a group represented by Formula (2a) below, and have different structures from each other.

### (Group represented by Formula (2a))

-Q¹-(X¹Q²)a-X²Q³ (2a)

Q¹ is an alkylene group or a haloalkylene group and a group to be attached to an atom M at position 13 in Formula (1) above.

Q¹ may be a linear alkylene group or a branched alkylene group. Q¹ is preferably a linear alkylene group.

The alkylene group for Q¹ has preferably 1 to 20 carbon atoms, more preferably 1 to 15 carbon atoms, further preferably 1 to 9 carbon atoms, and particularly preferably 2 to 6 carbon atoms.

When Q¹ is a haloalkylene group, at least one selected from the group consisting of I, Cl, Br, and F may be used as a halogen atom. The halogen atom is preferably at least one of C1 or F and more preferably F.

Q² is an alkylene group or a haloalkylene group, and preferred aspects of a number of carbon numbers in the alkylene group and the halogen atom are the same as for Q¹. The number of carbon atoms in the alkylene group for Q² may be the same as or different from that in the alkylene group for Q¹.

Q³ is an alkyl group or a haloalkyl group, and the alkyl group has preferably 1 to 20 carbon atoms, more preferably 1 to 12 carbon atoms, further preferably 1 to 7 carbon atoms, and particularly preferably 1 to 3 carbon atoms. Q³ may be a linear alkyl group or a branched alkyl group. Q³ is preferably a linear alkyl group.

When Q³ is a haloalkyl group, at least one selected from the group consisting of I, Cl, Br, and F may be used as a halogen atom. The halogen atom is preferably at least one of C1 or F and more preferably F.

X¹ and X² are each independently O, S, NR⁷⁰⁰, PR⁷⁰¹, or P (=O). X¹ and X² are each preferably O, S, or NR⁷⁰⁰, more preferably O or S, and further preferably O.

R⁷⁰⁰ is a hydrogen atom, an alkyl group having 1 to 20 carbon atoms, a cycloalkyl group having 3 to 8 carbon atoms, an aryl group having 5 to 12 carbon atoms, or a heteroaryl group having 3 to 12 carbon atoms. R⁷⁰⁰ is preferably a hydrogen atom, an alkyl group having 1 to 10 carbon atoms, or an aryl group having 5 to 12 carbon atoms. The alkyl group is preferably a methyl group, an ethyl group, a propyl group, a butyl group, or a pentyl group. The aryl group is preferably a phenyl group or a naphthyl group.

R⁷⁰¹ is a hydrogen atom, an alkyl group having 1 to 20 carbon atoms, a cycloalkyl group having 3 to 8 carbon atoms, an aryl group having 5 to 12 carbon atoms, or a heteroaryl group having 3 to 12 carbon atoms. R⁷⁰¹ is preferably a hydrogen atom, an alkyl group having 1 to 10 carbon atoms, or an aryl group having 5 to 12 carbon atoms. The alkyl group is preferably a methyl group, an ethyl group, a propyl group, a butyl group, or a pentyl group. The aryl group is preferably a phenyl group or a naphthyl group.

a is an integer of 0 or more and 10 or less, preferably an integer of 0 or more and 5 or less, more preferably an integer of 0 or more and 3 or less, and further preferably an integer of 0 or more and 2 or less.

The group represented by Formula (2a) is preferably a group in which X¹ and X² are oxygen atoms, as shown in Formula (2b) below.

-Q¹-(OQ²)a-OQ³ (2b)

Specific examples of Formula (2a) above include -CH₂OCH₃, -CH₂SCH₃, -CH₂CH₂OCH₃, -CH₂CH₂OCH₂CH₃, -CH₂CH₂SCH₃, -CH₂CH₂CH₂OCH₃, -CH₂CH₂CH₂SCH₃, -CH₂CH₂OCH₂CH₂OCH₃, -CH₂CH₂OCH₂CH₂OCH₂CH₃, - CH₂CH₂OCH₂CH₂OCH₂CH₂OCH₃, or -CH₂CH₂OCH₂CH₂OCH₂CH₂OCH₂CH₂OCH₃.

A number of carbon atoms in the alkyl group for R² can be equal to or larger than a number of carbon atoms in the alkyl group for R¹. In other words, if R¹ is a linear alkyl group, R² is a linear alkyl group having a larger number of carbon atoms than in R¹, or a branched alkyl group in which a number of carbon atoms is equal to or larger than in R¹.

If R¹ is a branched alkyl group, R² is a linear alkyl group in which a number of carbon atoms is equal to or larger than in R¹ or a branched alkyl group in which a number of carbon atoms is equal to or larger than in R¹ and which has a different structure from R¹.

If both R¹ and R² are branched alkyl groups each having 4 or more carbon atoms, a number of carbon atoms in a main chain of R¹ may be larger than a number of carbon atoms in a main chain of R². When they have the same number of carbon atoms in their main chains, a branched alkyl group in which an alkyl group branched from the main chain is substituted at a position closer to M in Formula (1) above is determined as R¹.

For example, when substituted alkyl groups substituted at M are an s-butyl group and an isobutyl group, the s-butyl group with a methyl group serving as a substituent being substituted at a position closer to M is determined as R¹ and the isobutyl group is determined as R².

R¹ preferably has 1 to 20 carbon atoms, more preferably 1 to 14 carbon atoms, further preferably 1 to 9 carbon atoms, particularly preferably 1 to 6 carbon atoms, and extremely preferably 1 to 3 carbon atoms.

R² preferably has 2 to 20 carbon atoms, more preferably 2 to 11 carbon atoms, further preferably 2 to 10 carbon atoms, particularly preferably 2 to 7 carbon atoms, and extremely preferably 2 to 4 carbon atoms.

A value obtained by subtracting a number of carbon atoms in the alkyl group for R¹ from a number of carbon atoms in the alkyl group for R² is preferably 1 to 15, more preferably 1 to 9, further preferably 1 to 5, and particularly preferably 1 to 3.

R¹ and R² are each independently preferably a linear or branched alkyl group having 1 to 20 carbon atoms, an alkyl group having 1 to 20 carbon atoms substituted with a halogen atom, or a group represented by Formula (2b), more preferably a linear or branched alkyl group having 1 to 10 carbon atoms, an alkyl group having 1 to 10 carbon atoms substituted with a halogen atom, or a group represented by Formula (2b) above, further preferably a linear alkyl group having 1 to 7 carbon atoms, an alkyl group having 1 to 7 carbon atoms substituted with a halogen atom, or a group represented by Formula (2b) above, and particularly preferably a linear alkyl group having 1 to 4 carbon atoms, an alkyl group having 1 to 4 carbon atoms substituted with a halogen atom, or a group represented by Formula (2b) above.

### (R⁵ and R⁶)

R⁵ and R⁶ are each independently a hydrogen atom or a substituent. R⁵ and R⁶ are each independently preferably a substituted or unsubstituted aryl group or a substituted or unsubstituted heteroaryl group.

R⁵ and R⁶ are each independently preferably a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or substituted or unsubstituted benzopyrrolinyl group. Furthermore, it is preferred that at least one of R⁵ or R⁶ be a substituted or unsubstituted phenyl group, and it is more preferred that both R⁵ and R⁶ be substituted phenyl groups.

A substituent that the phenyl group has is preferably a group represented by Formula (2a) above, a hydroxy group, an alkyl group, a haloalkyl group, a cycloalkyl group, an alkoxy group, an amino group, a heterocyclic group, a cyano group, a halogen atom, an alkylthio group, an arylthio group, a nitro group, a formyl group, a hydroxycarbonyl group, an alkylcarbonyl group, an alkoxycarbonyl group, an aralkyl group, an aralkoxy group, an aryloxy group, an aryl group, a heteroaryl group, a thiol group, an alkoxyalkylthio group, a haloalkylthio group, a cycloalkylthio group, a silyl group, an oxysilyl group, or a group represented by Formula (X3) described below. The substituent is more preferably an alkyl group, a haloalkyl group, an alkoxy group, an amino group, a heterocyclic group, a cyano group, a halogen atom, an alkylthio group, an arylthiol group, an aryloxy group, an aryl group, a heteroaryl group, an alkoxyalkylthio group, a haloalkylthio group, a silyl group, an oxysilyl group, or a group represented by Formula (X3) described below.

Specific examples thereof may be the same as those exemplified for R³ and R⁴ as described below.

### <Suitable first photochromic compound>

A first photochromic compound preferably has a backbone represented by Formula (4) below.

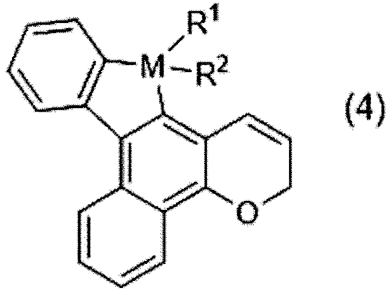

In Formula (4), R¹, R², and M are each the same as for Formula (1).

### <More suitable first photochromic compound>

A first photochromic compound is more preferably a photochromic compound represented by Formula (5) below. In Formula (5), R¹, R², and M are each the same as for Formula (1).

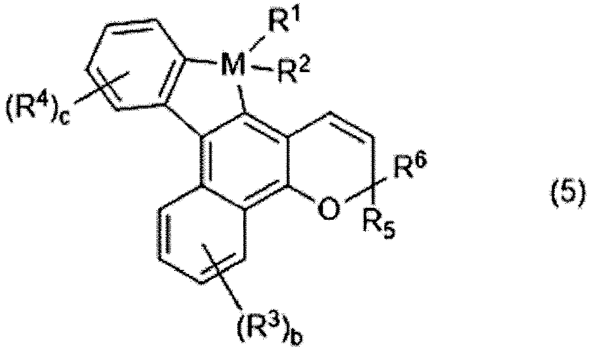

### (R³ and R⁴)

R³ and R⁴ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) described below, or a group represented by Formula (X3) described below.

b is an integer of 0 or more and 4 or less. When b is an integer of 2 or more and 4 or less, a plurality of R³s may be the same as or different from each other. When b is an integer of 2 or more and 4 or less and adjacent R³s are present, two adjacent R³s may be taken together with a carbon atom to which R³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings. A substituent the ring has is, for example, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group having 3 to 8 atoms, a cyano group, a nitro group, or a halogen atom. A combination of the adjacent R³s may be at positions 5 and 6, positions 6 and 7, or positions 7 and 8 of a naphthopyran backbone.

c is an integer of 0 or more and 4 or less. When c is an integer of 2 or more and 4 or less, a plurality of R⁴s may be the same as or different from each other. When c is an integer of 2 or more and 4 or less and adjacent R⁴s are present, two adjacent R⁴s may be taken together with a carbon atom to which R⁴s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings. A substituent the ring has is, for example, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group having 3 to 8 atoms, a cyano group, a nitro group, or a halogen atom. A combination of the adjacent R⁴s may be at positions 9 and 10, positions 10 and 11, or positions 11 and 12 of the naphthopyran backbone.

A ring having 5 to 8 atoms including a carbon atom to which R³ or R⁴ is attached may be formed. The ring may have a substituent and the substituent includes a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Specific examples of the substituent will be described below.

A suitable ring includes a ring represented by Formula (X5) below.

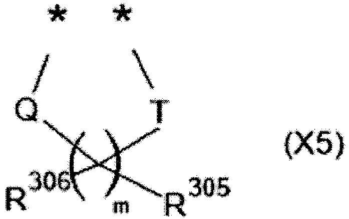

In Formula (X5) above, Q and T are each independently a sulfur atom, a substituted or unsubstituted methylene group, an oxygen atom, or a group represented by NR³⁰⁷. R³⁰⁷ is a hydrogen atom, a hydroxy group, an alkyl group, a haloalkyl group, a cycloalkyl group, an alkoxy group, a substituted or unsubstituted aryl group, a substituted or unsubstituted heteroaryl group, or a group represented by Formula (2a) above.

R³⁰⁵ and R³⁰⁶ are each independently preferably a hydroxy group, an alkyl group, a haloalkyl group, a cycloalkyl group, an alkoxy group, an amino group, a substituted amino group, a substituted or unsubstituted heterocyclic group, a cyano group, a nitro group, a formyl group, a hydroxycarbonyl group, an alkylcarbonyl group, an alkoxycarbonyl group, a halogen atom, a substituted or unsubstituted aralkyl group, a substituted or unsubstituted aralkoxy group, a substituted or unsubstituted aryl group, a thiol group, an alkylthio group, an alkoxyalkylthio group, a haloalkylthio group, a cycloalkylthio group, or a substituted or unsubstituted arylthio group.

In addition, R³⁰⁵ and R³⁰⁶ may be taken together with a carbon atom to which they are attached to form a substituted or unsubstituted aliphatic ring. Specific examples of the aliphatic ring include a cyclopentane ring, a cyclohexane ring, etc. For the aliphatic ring, 1 to 8 hydrogen atoms, particularly preferably 1 to 4 hydrogen atoms may be substituted with at least one group selected from the group consisting of a hydroxy group, an alkyl group, a haloalkyl group, a cycloalkyl group, an alkoxy group, an amino group, a substituted amino group, a heterocyclic group, a cyano group, a nitro group, and a halogen atom. Specific examples of the substituent will be described below. In the Formula, m is an integer of 1 or more and 4 or less.

### (Group represented by Formula (X))

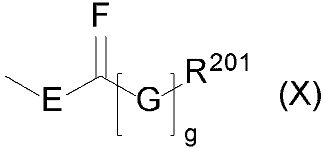

In Formula (X), E is an oxygen atom or a group represented by NR¹⁰¹. R¹⁰¹ is a hydrogen atom or an alkyl group. E is preferably a group represented by NR¹⁰¹ in which R¹⁰¹ is a hydrogen atom or an alkyl group having 1 to 6 carbon atoms.

F is an oxygen atom or a sulfur atom. F is preferably an oxygen atom.

G is an oxygen atom, a sulfur atom, or a group represented by NR²⁰². R²⁰² is a hydrogen atom, an alkyl group, a cycloalkyl group, an aryl group, or a heteroaryl group. G is preferably NH.

g is 0 or 1.

R²⁰¹ is a hydrogen atom, an alkyl group, a cycloalkyl group, an aryl group, or a heteroaryl group. When G is an oxygen atom or a sulfur atom, R²⁰¹ is a group other than a hydrogen atom. R²⁰¹ is preferably an alkyl group having 1 to 6 carbon atoms, an alkoxy group having 1 to 6 carbon atoms, or an aryl group having 6 to 12 carbon atoms.

Suitable groups represented by Formula (X) are as follows.

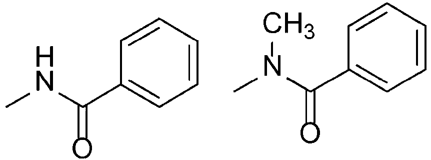

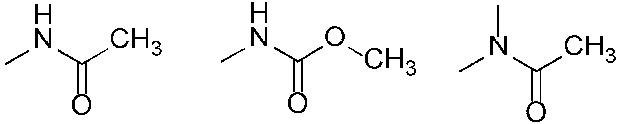

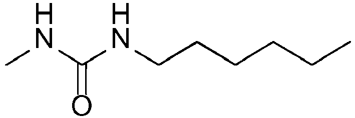

### (Group represented by Formula (X3))

L¹-R⁴⁰⁰ (X3)

In Formula (X3) above, R⁴⁰⁰ is a hydrogen atom, an alkyl group, an aryl group, a polymerizable group, a photochromic group, or a silyl group having an alkyl group, an alkoxy group, or an aryl group as a substituent.

L¹ is a group represented by Formula (X2) below:

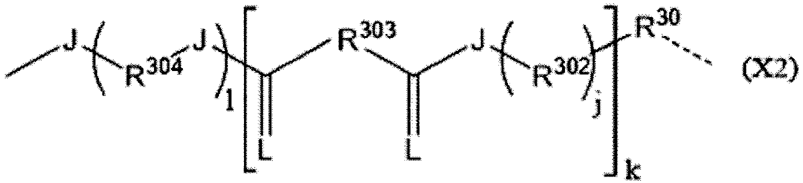

In Formula (X2), R³⁰ is a group represented by Formula (X2a) below.

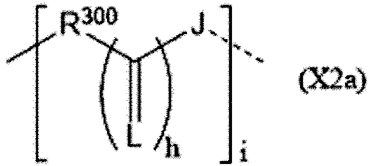

In Formulae (X2) and (X2a) above, J is a divalent group. A plurality of Js are each independently directly attached, a substituted or unsubstituted methylene group, an oxygen atom, a sulfur atom, or NR³⁰¹. R³⁰¹ is a hydrogen atom or an alkyl group. R³⁰¹ is preferably an alkyl group having 1 to 20 carbon atoms. The alkyl group preferably has a silyl group having an alkyl group having 1 to 10 carbon atoms, a polymerizable group, or a photochromic group as a substituent.

Examples of the polymerizable group include a radical polymerizable group such as a vinyl group, a 1-chlorovinyl group, an allyl group, a styryl group, a (meth)acryl group, a 2-(methacryloxy)ethylcarbamyl group, a 2-(methacryloxy)ethoxycarbonyl group, or a crotyl group. In addition to the above-mentioned groups, an epoxy group, an episulfide group, a thietanyl group, an OH group, an SH group, an NH₂ group, a COOH group, an NCO group, an NCS group, etc., may also be used. The polymerizable group is preferably at least one selected from the group consisting of a (meth)acrylic group, a 2-(methacryloxy)ethylcarbamyl group, a 2-(methacryloxy)ethoxycarbonyl group, an epoxy group, an OH group, an SH group, an NH₂ group, and a COOH group.

The photochromic group is a group containing a photochromic moiety. The photochromic group is representatively exemplified by naphthopyran, spirooxazine, spiropyran, fulgide, fulgimide, or diarylethene. Indenonaphthopyran is preferred from the viewpoint of its ability to develop an excellent photochromic property, and indeno[2,1-f]naphtho[1,2-b]pyran is particularly preferred.

Indeno[2,1-f]naphtho[1,2-b]pyran is preferably a group represented by Formula (X4) below:

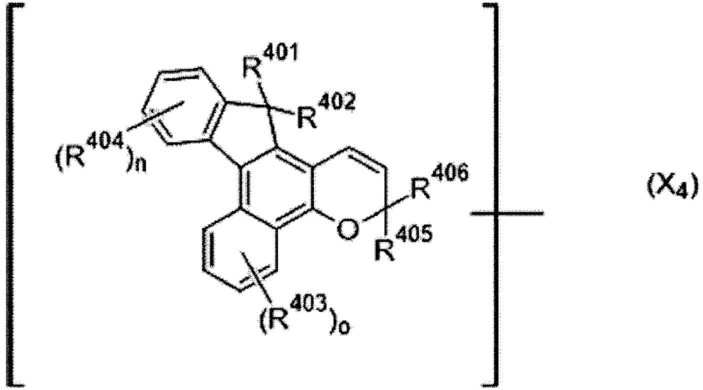

In Formula (X4) above, R⁴⁰¹ and R⁴⁰² may be the same as groups to be used for R³ and R⁴ as described above.

R⁴⁰³ and R⁴⁰⁴ each independently may be the same as groups to be used for R³ and R⁴ as described above.

R⁴⁰⁵ and R⁴⁰⁶ are each independently a substituted or unsubstituted aryl group or a substituted or unsubstituted heteroaryl group. R⁴⁰⁵ and R⁴⁰⁶ may be the same as groups to be used for R⁵ and R⁶ as described below.

o is an integer of 0 or more and 4 or less. n is an integer of 0 or more and 4 or less. When o is an integer of 2 or more and 4 or less, a plurality of R⁴⁰³s may be the same as or different from each other. When n is an integer of 2 or more and 4 or less, a plurality of R⁴⁰⁴s may be the same as or different from each other.

At least one of substituents on the aryl group or the heteroaryl group for R⁴⁰¹, R⁴⁰², R⁴⁰³, R⁴⁰⁴, or R⁴⁰⁵ is attached to L¹.

L is an oxygen atom or a sulfur atom.

R³⁰⁰ is an alkylene group, or a silylene group having an alkyl group or an aryl group as a substituent. R³⁰⁰ is preferably an alkylene group having 1 to 6 carbon atoms or a silylene group having an alkyl group having 1 to 6 carbon atoms as a substituent.

R³⁰², R³⁰³, and R³⁰⁴ are each independently an alkylene group. R³⁰² is preferably an alkylene group having 1 to 6 carbon atoms. R³⁰³ is preferably an alkylene group having 1 to 6 carbon atoms. R³⁰⁴ is preferably an alkylene group having 1 to 6 carbon atoms.

h, j, k, and l are each independently 0 or 1.

i is an integer of 0 or more and 200 or less. A plurality of groups each with a subscript i may have structures identical to or different from each other. i is preferably an integer of 5 or more and 100 or less, more preferably 8 or more and 75 or less, and further preferably 10 or more and 70 or less.

A dashed line represents a bond to R⁴⁰⁰.

A particularly suitable group represented by Formula (X2) above is represented by any of the following formulae:

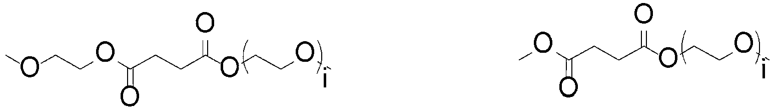

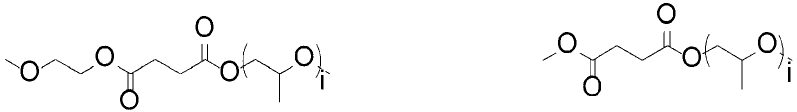

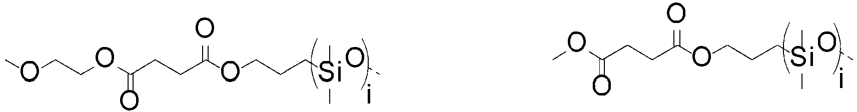

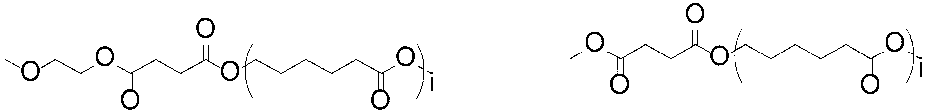

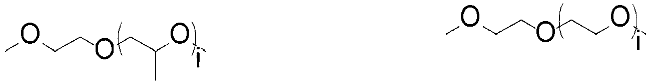

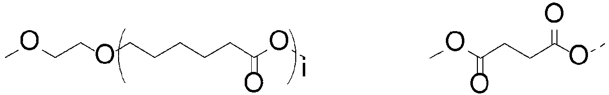

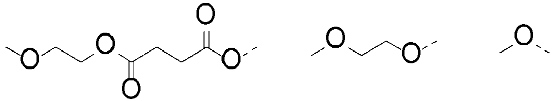

### (Specific examples of R³ and R⁴)

The alkyl group is preferably a linear or branched alkyl group having 1 to 20 carbon atoms, more preferably a linear alkyl group having 1 to 10 carbon atoms, and further preferably a linear alkyl group having 1 to 6 carbon atoms. Specific examples of the alkyl group include a methyl group, an ethyl group, a propyl group, a butyl group, a pentyl group, or a hexyl group.

The haloalkyl group is preferably a haloalkyl group having 1 to 6 carbon atoms. A number of halogen atoms is preferably 1 or more and 10 or less and more preferably 2 or more and 5 or less. The haloalkyl group having 1 to 6 carbon atoms is preferably an alkyl group substituted with a fluorine atom, a chlorine atom, or a bromine atom. The haloalkyl group preferably has a terminal perfluoromethyl group. Suitable examples of the haloalkyl group include a trifluoromethyl group, a trifluoroethyl group, a trifluoropropyl group, a tetrafluoroethyl group, a chloromethyl group, a 2-chloroethyl group, a bromomethyl group, etc.

The cycloalkyl group is preferably a cycloalkyl group having 3 to 8 carbon atoms (a cycloalkyl group in which 3 to 8 carbon atoms form a ring). Examples of the cycloalkyl group having 3 to 8 carbon atoms include a cyclopropyl group, a cyclobutyl group, a cyclopentyl group, a cyclohexyl group, etc. Note that, the cycloalkyl group may have a substituent, but a number of carbon atoms (3 to 8 carbon atoms) shall not include a number of carbon atoms on the substituent. Examples of the substituent that the cycloalkyl group may have include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The alkoxy group is preferably a linear or branched alkoxy group having 1 to 20 carbon atoms, more preferably an alkoxy group having 1 to 10 carbon atoms, and further preferably an alkoxy group having 1 to 6 carbon atoms. Suitable examples of the alkoxy group having 1 to 6 carbon atoms include a methoxy group, an ethoxy group, an n-propoxy group, an isopropoxy group, an n-butoxy group, a sec-butoxy group, a tert-butoxy group, etc.

The amino group may be a primary amino group (-NH₂), or a secondary or tertiary amino group with one or two hydrogen atoms being substituted. Examples of a substituent that the substituted amino group has include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc. Suitable examples of the amino group include an amino group, a methylamino group, a dimethylamino group, an ethylamino group, a diethylamino group, a methylphenylamino group, a diphenylamino group, etc.

The heterocyclic group preferably has 3 to 10 atoms. A heteroatom in the heterocyclic group is preferably at least one selected from the group consisting of an oxygen atom, a nitrogen atom, a sulfur atom, and a phosphorus atom. A number of the heteroatoms is, for example, 1 or more and 5 or less and preferably 1 or 2. Specific examples of the heterocyclic group include, for example, an aliphatic heterocyclic group such as a morpholino group, a piperidino group, a pyrrolidinyl group, a piperazino group, or an N-methylpiperazino group; an aromatic heterocyclic group such as an indolinyl group, etc. The heterocyclic group may be a 2,6-dimethylmorpholino group, a 2,6-dimethylpiperidino group, or a 2,2,6,6-tetramethylpiperidino group. Note that, the heterocyclic group may have a substituent, but a number of atoms (3 to 10 atoms) shall not include a number of atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

Examples of a halogen atom include a fluorine atom, a chlorine atom, a bromine atom, or an iodine atom.

The alkylthio group is preferably an alkylthio group having 1 to 6 carbon atoms. Examples of the alkylthio group having 1 to 6 carbon atoms include a methylthio group, an ethylthio group, an n-propylthio group, an isopropylthio group, an n-butylthio group, a sec-butylthio group, a t-butylthio group, etc.

The arylthio group is preferably an arylthio group having 6 to 10 carbon atoms. Examples of the arylthio group having 6 to 10 carbon atoms include a phenylthio group, a 1-naphthylthio group, a 2-naphthylthio group, etc. Note that, the arylthio group may have a substituent, but a number of carbon atoms (6 to 10 carbon atoms) shall not include a number of carbon atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The alkylcarbonyl group is preferably an alkylcarbonyl group having 2 to 7 carbon atoms. Examples of the alkylcarbonyl group having 2 to 7 carbon atoms include an acetyl group, an ethylcarbonyl group, etc.

The alkoxycarbonyl group is preferably an alkoxycarbonyl group having 2 to 7 carbon atoms. Examples of the alkoxycarbonyl group having 2 to 7 carbon atoms include a methoxycarbonyl group, an ethoxycarbonyl group, etc.

The aralkyl group is preferably an aralkyl group having 7 to 11 carbon atoms. Examples of the aralkyl group having 7 to 11 carbon atoms include a benzyl group, a phenylethyl group, a phenylpropyl group, a phenylbutyl group, a naphthylmethyl group, etc. Note that, the aralkyl group may have a substituent, but a number of carbon atoms (7 to 11 carbon atoms) shall not include a number of carbon atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The aralkoxy group is preferably an aralkoxy group having 7 to 11 carbon atoms. Examples of the aralkoxy group having 7 to 11 carbon atoms include a benzyloxy group, a naphthylmethoxy group, etc. Note that, the aralkoxy group may have a substituent, but a number of carbon atoms (7 to 11 carbon atoms) shall not include a number of carbon atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The aryloxy group is preferably an aryloxy group having 6 to 12 carbon atoms. Examples of the aryloxy group having 6 to 12 carbon atoms include a phenyloxy group, a naphthyloxy group, etc. Note that, the aryloxy group may have a substituent, but a number of carbon atoms (6 to 12 carbon atoms) shall not include a number of carbon atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The aryl group is not particularly limited, and is preferably an aryl group having 5 to 12 carbon atoms. Examples of the aryl group having 5 to 12 carbon atoms include a phenyl group, a 1-naphthyl group, a 2-naphthyl group, etc. Note that, the aryl group may have a substituent, but a number of carbon atoms (5 to 12 carbon atoms) shall not include a number of carbon atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The heteroaryl group is not particularly limited, and is preferably a heteroaryl group having 3 to 12 carbon atoms. A heteroatom may be at least one selected from the group consisting of an oxygen atom, a sulfur atom, a nitrogen atom, and a phosphorus atom. A number of the heteroatoms in the heteroaryl group is, for example, 1 or more and 3 or less and preferably 1 or 2. A number of the carbon atoms in the heteroaryl group is, for example, 4 to 11 and preferably 5 to 9. Note that, the heteroaryl group may have a substituent, but a number of carbon atoms (3 to 12 carbon atoms) shall not include a number of carbon atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

Examples of the heteroaryl group having 3 to 12 carbon atoms include a thienyl group, a furyl group, a pyrrolinyl group, a pyridyl group, a benzothienyl group, a benzofuranyl group, a benzopyrrolinyl group, etc.

The alkoxyalkylthio group is preferably an alkoxyalkylthio group having 2 to 9 carbon atoms. Examples of the alkoxyalkylthio group having 2 to 9 carbon atoms include a methoxymethylthio group, a methoxyethylthio group, a methoxy n-propylthio group, a methoxy n-butylthio group, an ethoxyethylthio group, an n-propoxypropylthio group, etc.

The haloalkylthio group is preferably a haloalkylthio group having 1 to 6 carbon atoms. Examples of the haloalkylthio group having 1 to 6 carbon atoms include a trifluoromethylthio group, a tetrafluoroethylthio group, a chloromethylthio group, a 2-chloroethylthio group, a bromomethylthio group, etc.

The cycloalkylthio group is preferably a cycloalkylthio group having 3 to 8 carbon atoms. Examples of the cycloalkylthio group having 3 to 8 carbon atoms include a cyclopropylthio group, a cyclobutylthio group, a cyclopentylthio group, a cyclohexylthio group, etc. Note that, the cycloalkylthio group may have a substituent, but a number of carbon atoms (3 to 8 carbon atoms) shall not include a number of carbon atoms on the substituent. Examples of the substituent include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The silyl group may have a substituent. A substituent that the silyl group has is not particularly limited, and includes an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

The oxysilyl group may have a substituent. The substituent that the oxysilyl group has is not particularly limited, and includes an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, a heteroaryl group having 4 to 14 carbon atoms, etc.

### (R⁵ and R⁶)

R⁵ and R⁶ are each independently a substituted or unsubstituted aryl group or a substituted or unsubstituted heteroaryl group.

R⁵ and R⁶ are each independently preferably a substituted or unsubstituted phenyl group, a substituted or unsubstituted 1-naphthyl group, a substituted or unsubstituted 2-naphthyl group, a substituted or unsubstituted thienyl group, a substituted or unsubstituted furyl group, a substituted or unsubstituted pyrrolinyl group, a substituted or unsubstituted pyridyl group, a substituted or unsubstituted benzothienyl group, a substituted or unsubstituted benzofuranyl group, or substituted or unsubstituted benzopyrrolinyl group. Furthermore, it is preferred that at least one of R⁵ or R⁶ be a substituted or unsubstituted phenyl group, and it is more preferred that both R⁵ and R⁶ be substituted phenyl groups.

A substituent that the phenyl group has is preferably a group represented by Formula (2a) above, a hydroxy group, an alkyl group, a haloalkyl group, a cycloalkyl group, an alkoxy group, an amino group, a heterocyclic group, a cyano group, a halogen atom, an alkylthio group, an arylthio group, a nitro group, a formyl group, a hydroxycarbonyl group, an alkylcarbonyl group, an alkoxycarbonyl group, an aralkyl group, an aralkoxy group, an aryloxy group, an aryl group, a heteroaryl group, a thiol group, an alkoxyalkylthio group, a haloalkylthio group, a cycloalkylthio group, a silyl group, an oxysilyl group, or a group represented by Formula (X3) above. The substituent is more preferably an alkyl group, a haloalkyl group, an alkoxy group, an amino group, a heterocyclic group, a cyano group, a halogen atom, an alkylthio group, an arylthio group, an aryloxy group, an aryl group, a heteroaryl group, an alkoxyalkylthio group, a haloalkylthio group, a silyl group, an oxysilyl group, or a group represented by Formula (X3) above.

Specific examples thereof may be the same as those exemplified for R³ and R⁴.

### <Particularly suitable first photochromic compound>

A particularly suitable first photochromic compound includes a photochromic compound represented by Formula (6) below. In Formula (6), R¹, R², R³, R⁴, b, and c are each the same as for Formula (5).

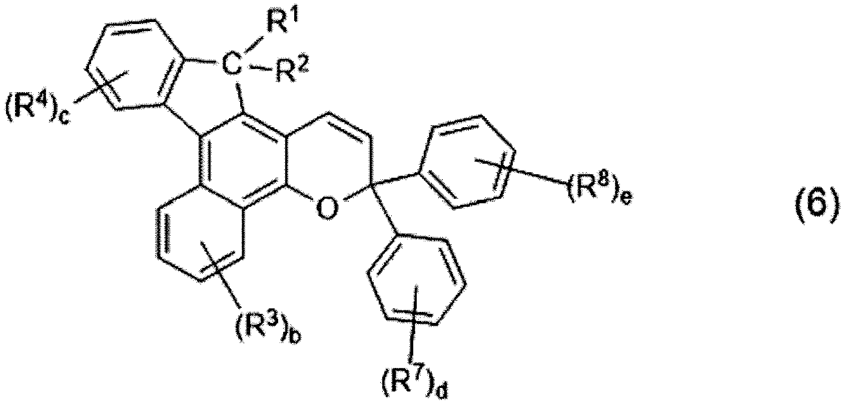

### (R⁷ and R⁸)

R⁷ and R⁸ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above. Specific examples thereof may be the same as those exemplified for R³ and R⁴.

d denotes a number of R⁷ and is an integer of 0 or more and 5 or less. When d is 2 or more, R⁷s may be the same as or different from each other.

When d is an integer of 2 or more and 5 or less and adjacent R⁷s are present, two adjacent R⁷s may be taken together with a carbon atom to which R⁷s are attached to form a ring optionally including at least one atom selected from the group consisting of an oxygen atom, a sulfur atom, a carbon atom, and a nitrogen atom and the ring may have a substituent. The substituent may be a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Note that, the ring can also simultaneously have two or more atoms selected from the group consisting of an oxygen atom, a sulfur atom, a carbon atom, and a nitrogen atom.

e denotes a number of R⁸ and is an integer of 0 or more and 5 or less. When e is 2 or more, R⁸s may be the same as or different from each other.

When e is an integer of 2 or more and 5 or less and adjacent R⁸s are present, two adjacent R⁸s may be taken together with a carbon atom to which R⁸s are attached to form a ring optionally including at least one atom selected from the group consisting of an oxygen atom, a sulfur atom, a carbon atom, and a nitrogen atom and the ring may have a substituent. The substituent may be a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Note that, the ring can also simultaneously have two or more atoms selected from the group consisting of an oxygen atom, a sulfur atom, a carbon atom, and a nitrogen atom.

For R⁷ and R⁸, each independently, two adjacent to each other may be taken together to form a ring group optionally including at least one selected from the group consisting of an oxygen atom, a sulfur atom, a carbon atom, and a nitrogen atom. This ring group is not particularly limited, and is preferably a ring having 5 to 8 atoms including a carbon atom to which R⁷ and R⁸ are attached. The ring may have a substituent. The substituent may be a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Specific examples of these substituents are the same groups as described above. For the ring, it is preferred that a plurality of the rings be taken together to form a ring represented by Formula (X5) above.

Specific examples of the first photochromic compound include the following compounds: in which Me denotes a methyl group and Pr denotes a propyl group.

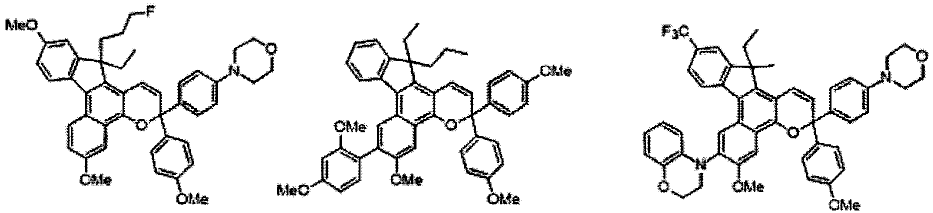

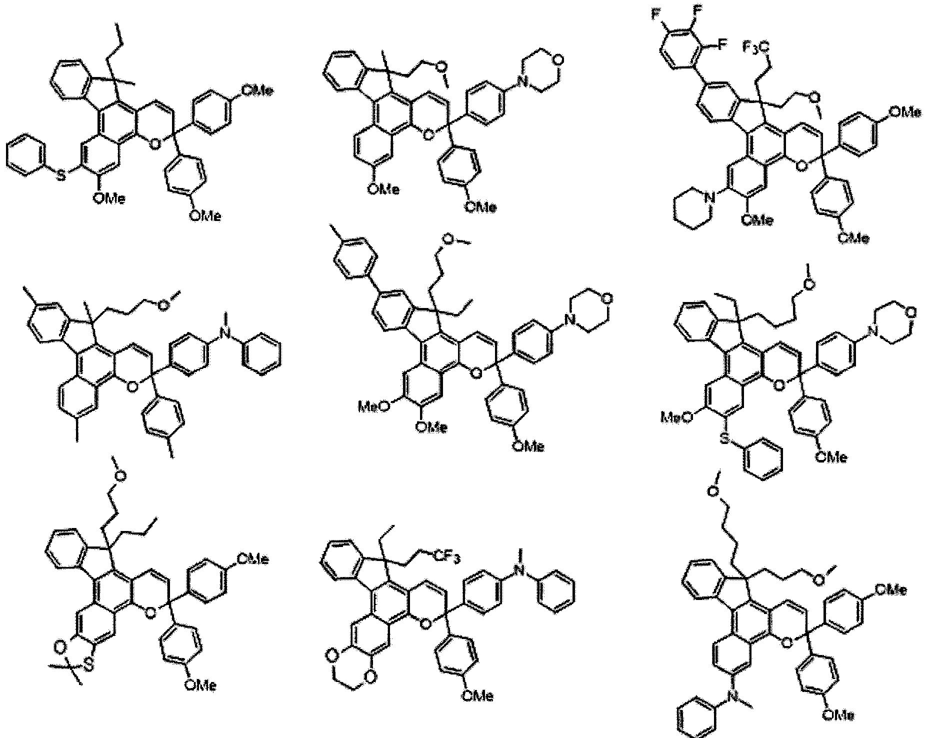

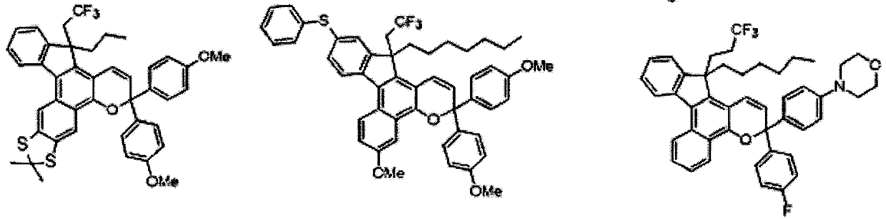

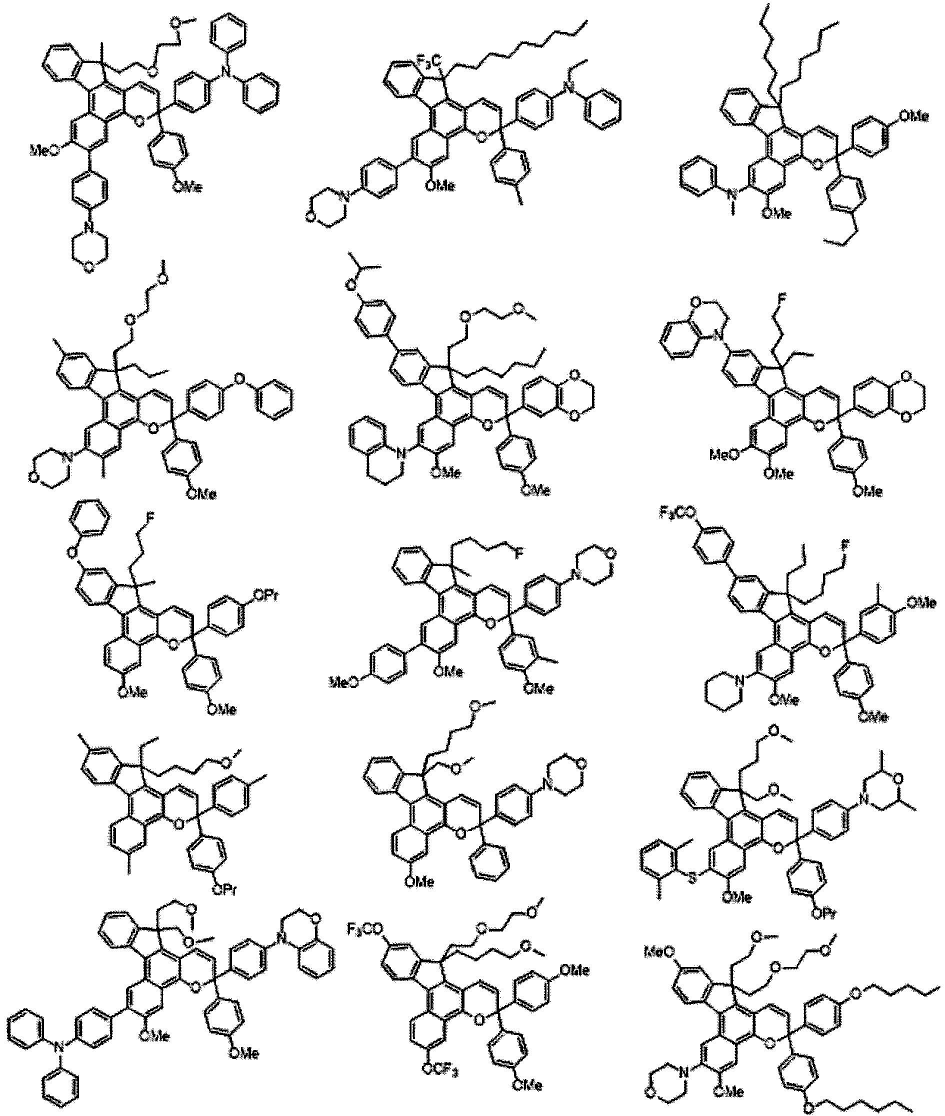

### <Second photochromic compound>

Suitable examples of a photochromic compound to be combined with a first photochromic compound include a second photochromic compound including a second photochromic compound A, a second photochromic compound B, and a second photochromic compound C. Only one type of the second photochromic compound may be included, or a combination of several types thereof may be included.

### <Second photochromic compound A>

A second photochromic composition A is a photochromic compound represented by Formula (1) above and having a different structure from a first photochromic compound. Suitable structure and substituent thereof may be the same as those described for the first photochromic compound.

### <Second photochromic compound B>

A second photochromic compound B is represented by Formula (2) below.

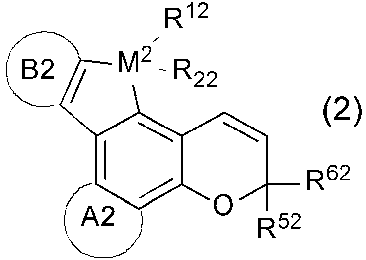

### (M²)

M² is C, Si, or Ge, with C preferred.

### (Ring A2 and Ring B2)

Ring A2 and Ring B2 are each independently a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. Suitable examples thereof may be the same as those exemplified for Ring A.

### (R¹² and R²²)

R¹² and R²² are each a substituted or unsubstituted alkyl group or a group represented by Formula (2a) above. R¹² and R²² have the same structure as each other. Suitable examples of R¹² and R²² may be the same as those exemplified for R¹.

### (R⁵² and R⁶²)

R⁵² and R⁶² are each independently a hydrogen atom or a substituent. Preferably, R⁵² and R⁶² are each independently a substituted or unsubstituted aryl group or a substituted or unsubstituted heteroaryl group. Suitable examples thereof may be the same as those exemplified for R⁵ and R⁶.

### <Suitable second photochromic compound B>

A second photochromic compound B is preferably a photochromic compound represented by Formula (7) below. In Formula (7), R¹² and R²² are each the same as for Formula (2).

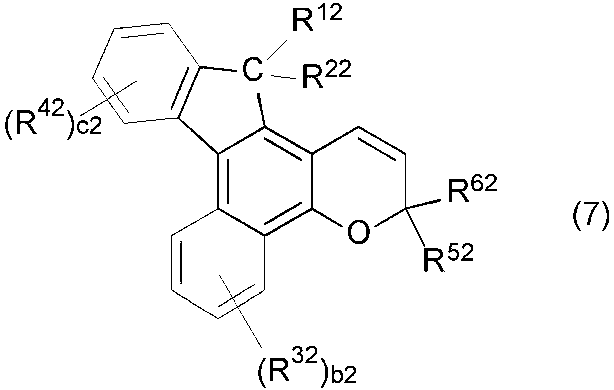

### (R³² and R⁴²)

R³² and R⁴² are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above. Suitable examples of R³² and R⁴² may be the same as those exemplified for R³ and R⁴.

b2 is an integer of 0 or more and 4 or less. When b2 is an integer of 2 or more and 4 or less, a plurality of R³²s may be the same as or different from each other. When b2 is an integer of 2 or more and 4 or less and adjacent R³²s are present, two adjacent R³²s may be taken together with a carbon atom to which R³²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings. The substituent the ring has is, for example, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group having 3 to 8 atoms, a cyano group, a nitro group, or a halogen atom. A combination of the adjacent R³²s may be at positions 5 and 6, positions 6 and 7, or positions 7 and 8 of the naphthopyran backbone.

c2 is an integer of 0 or more and 4 or less. When c2 is an integer of 2 or more and 4 or less, a plurality of R⁴²s may be the same as or different from each other. When c2 is an integer of 2 or more and 4 or less and adjacent R⁴²s are present, two adjacent R⁴²s may be taken together with a carbon atom to which R⁴²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings. The substituent the ring has is, for example, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group having 3 to 8 atoms, a cyano group, a nitro group, or a halogen atom. A combination of the adjacent R⁴²s may be at positions 9 and 10, positions 10 and 11, or positions 11 and 12 of the naphthopyran backbone.

A ring having 5 to 8 atoms including a carbon atom to which R³² or R⁴² is attached may be formed, and a suitable ring includes a ring represented by Formula (X5) above.

### <Particularly suitable second photochromic compound B>

A second photochromic compound B is particularly preferably a photochromic compound represented by Formula (8) below. In Formula (8), R¹², R²², R³², R⁴², b2, and c2 are each the same as for Formula (7) above.

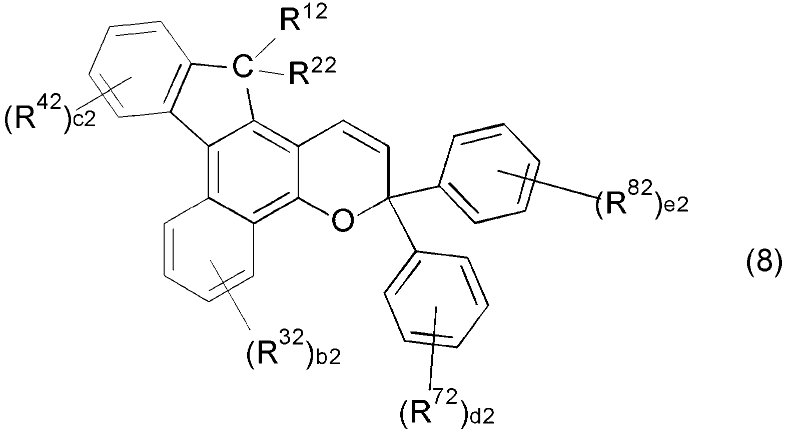

### (R⁷² and R⁸²)

R⁷² and R⁸² are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above. Suitable examples of R⁷² and R⁸² may be the same as those exemplified for R³ and R⁴.

d2 denotes a number of R⁷² and is an integer of 0 or more and 5 or less. When d2 is 2 or more, R⁷²s may be the same as or different from each other.

When d2 is an integer of 2 or more and 5 or less and adjacent R⁷²s are present, two adjacent R⁷²s may be taken together with a carbon atom to which R⁷²s are attached to form a ring optionally including at least one atom selected from the group consisting of an oxygen atom, a sulfur atom, a carbon atom, and a nitrogen atom and the ring may have a substituent. Note that, the ring can also simultaneously have two or more atoms selected from the group consisting of an oxygen atom, a sulfur atom, a carbon atom, and a nitrogen atom.

e2 denotes a number of R⁸² and is an integer of 0 or more and 5 or less. When e2 is 2 or more, R⁸²s may be the same as or different from each other.

When e2 is an integer of 2 or more and 5 or less and adjacent R⁸²s are present, two adjacent R⁸²s may be taken together with a carbon atom to which R⁸²s are attached to form a ring optionally including at least one atom selected from the group consisting of an oxygen atom, a sulfur atom, a carbon atom, and a nitrogen atom and the ring may have a substituent. Note that, the ring can also simultaneously have two or more atoms selected from the group consisting of an oxygen atom, a sulfur atom, a carbon atom, and a nitrogen atom.

For R⁷² and R⁸², each independently, two adjacent to each other may be taken together to form a ring group optionally including at least one selected from the group consisting of an oxygen atom, a sulfur atom, a carbon atom, and a nitrogen atom. This ring group is not particularly limited, and is preferably a ring having 5 to 8 atoms including a carbon atom to which R⁷² and R⁸² are attached. The ring may have a substituent. The substituent may be a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Specific examples of these substituents are the same groups as described above. For the ring, it is preferred that a plurality of the rings be taken together to form a ring represented by Formula (X5) above.

Specific examples of the second photochromic compound B include the following compounds: in which Me denotes a methyl group and Pr denotes a propyl group.

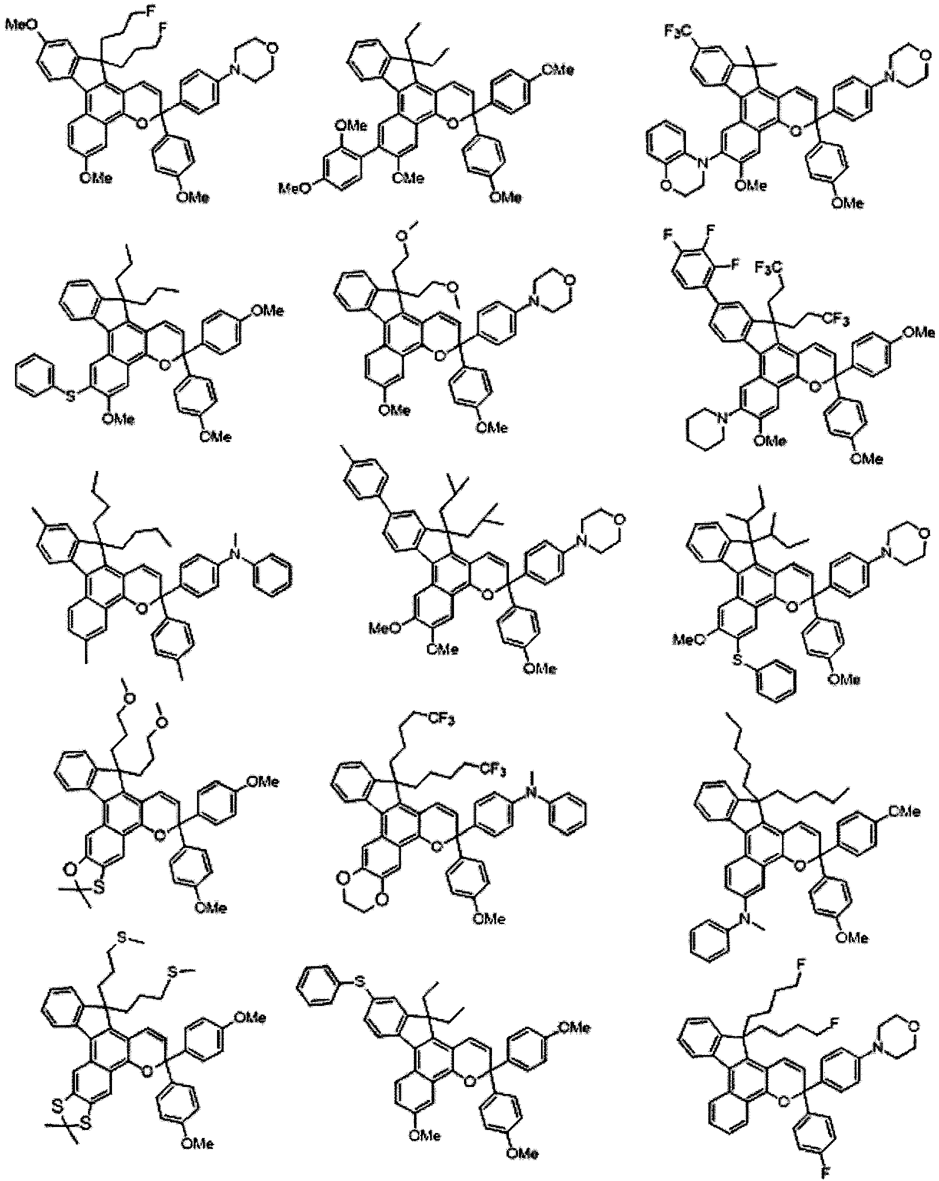

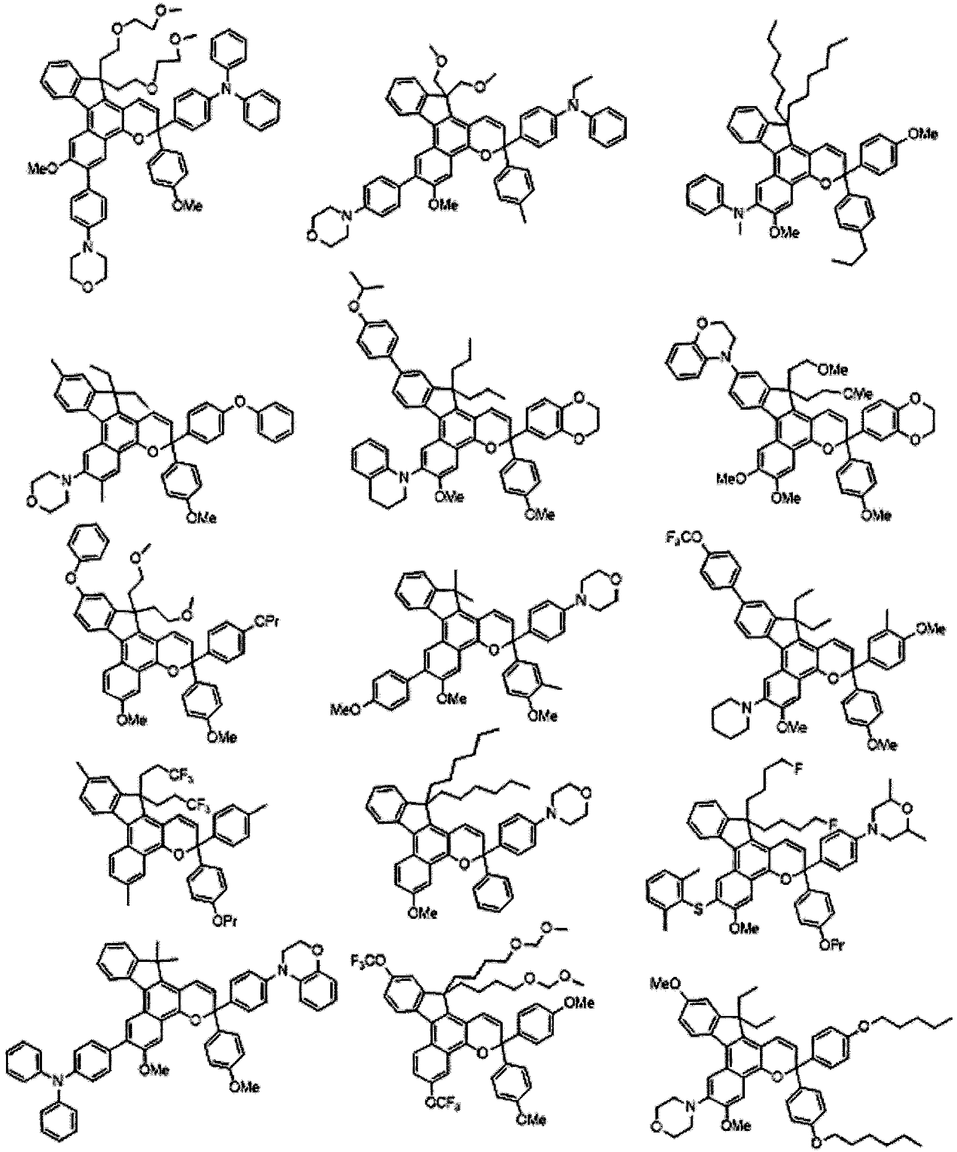

The second photochromic compound B can be synthesized by any of known methods.

### <Second photochromic compound C>

A second photochromic compound C is represented by Formula (3) below.

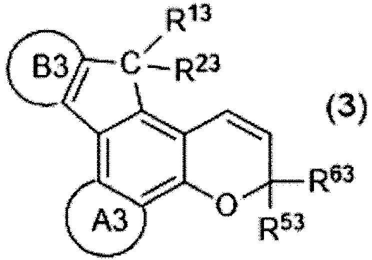

### (Ring A3 and Ring B3)

Ring A3 and Ring B3 are each independently a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. Suitable examples thereof include the same as those exemplified for Ring A.

### (R¹³ and R²³)

R¹³ and R²³ are taken together with a carbon atom to which they are attached to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the above-mentioned aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the above-mentioned heterocyclic ring. The substituent the ring has is, for example, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group having 3 to 8 atoms, a cyano group, a nitro group, or a halogen atom.

A number of ring member carbon atoms in the aliphatic ring formed by R¹³ and R²³ is more preferably from 5 to 16 and further preferably from 6 to 10. Specific examples of the aliphatic ring include a cyclopentane ring, a cyclohexane ring, a cyclooctane ring, a cycloheptane ring, a norbornane ring, a bicyclononane ring, an adamantane ring, a spirodicyclohexane ring, etc. The aliphatic ring is preferably a cyclopentane ring, a cyclohexane ring, a cycloheptane ring, a cyclooctane ring, a cyclononane ring, a cyclodecane ring, a cycloundecane ring, a cyclododecane ring, or a spirodicyclohexane ring.

A number of ring member carbon atoms in the fused polycyclic ring is preferably 3 to 20 and more preferably 5 to 10. Specific examples of the fused polycyclic ring include a phenanthrene ring.

A number of ring member atoms in the heterocyclic group is preferably 3 to 20. Specific examples of the heterocyclic ring include a thiophene ring, a furan ring, a pyridine ring, etc.

A number of ring member atoms in the heterocyclic aromatic ring is preferably 5 to 16. Specific examples of the heterocyclic aromatic ring include a phenylfuran ring, a biphenylthiophene ring, etc.

Such an aliphatic ring, fused polycyclic ring, heterocyclic ring, or heterocyclic aromatic ring may have a substituent. Examples of the substituent include at least one substituent selected from the group consisting of an alkyl group, a haloalkyl group, a cycloalkyl group, an alkoxy group, an amino group, a substituted amino group, and a halogen atom. The substituent is preferably at least one substituent selected from the group consisting of an alkyl group, a cycloalkyl group, a haloalkyl group, and an alkoxy group. A number of the substituent is 0 or more and 10 or less and preferably 2 or more and 4 or less.

Specifically, the ring formed by R¹³ and R²³ is further preferably a ring selected from a cyclopentane ring, a cyclohexane ring, a cycloheptane ring, a cyclooctane ring, a cyclononane ring, a cyclodecane ring, a cycloundecane ring, a cyclododecane ring, or a spirodicyclohexane ring. Examples of more suitable groups may include groups represented by formulae below. Note that, a carbon atom marked with * in the formulae represents a carbon atom to which R¹³ and R²³ are attached.

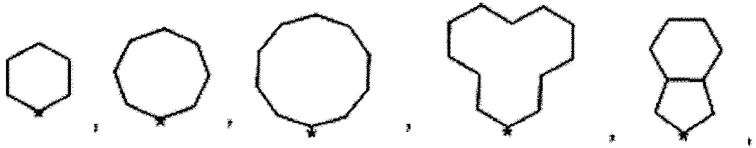

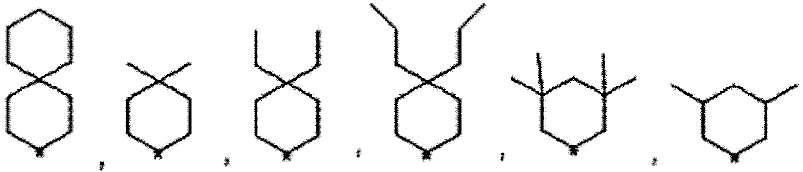

### (R⁵³ and R⁶³)

R⁵³ and R⁶³ are each a hydrogen atom or a substituent. Preferably, R⁵³ and R⁶³ are each independently a substituted or unsubstituted aryl group or a substituted or unsubstituted heteroaryl group. Suitable examples thereof may be the same as groups exemplified for R⁵ and R⁶.

<Suitable second photochromic compound C>

A second photochromic compound C is preferably a photochromic compound represented by Formula (9) below. In Formula (9), R¹³ and R²³ are each the same as for Formula (3) above.

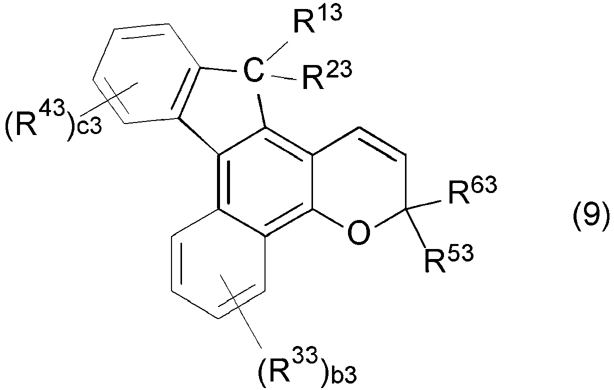

### (R³³ and R⁴³)

R³³ and R⁴³ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above. Suitable examples of R³³ and R⁴³ may be the same as those exemplified for R³ and R⁴.

b3 is an integer of 0 or more and 4 or less. When b3 is an integer of 2 or more and 4 or less, a plurality of R³³s may be the same as or different from each other. When b3 is an integer of 2 or more and 4 or less and adjacent R³³s are present, two adjacent R³³s may be taken together with a carbon atom to which R³³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings. The substituent the ring has is, for example, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group having 3 to 8 atoms, a cyano group, a nitro group, or a halogen atom. A combination of the adjacent R³³s may be at positions 5 and 6, positions 6 and 7, or positions 7 and 8 of the naphthopyran backbone.

c3 is an integer of 0 or more and 4 or less. When c3 is an integer of 2 or more and 4 or less, a plurality of R⁴³s may be the same as or different from each other. When c3 is an integer of 2 or more and 4 or less and adjacent R⁴³s are present, two adjacent R⁴³s may be taken together with a carbon atom to which R⁴³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings. The substituent the ring has is, for example, a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group having 3 to 8 atoms, a cyano group, a nitro group, or a halogen atom. A combination of the adjacent R⁴³s may be at positions 9 and 10, positions 10 and 11, or positions 11 and 12 of the naphthopyran backbone.

A ring having 5 to 8 atoms including a carbon atom to which R³³ or R⁴³ is attached may be formed, and a suitable ring includes a ring represented by Formula (X5) above.

### <Particularly suitable second photochromic compound C>

A second photochromic compound C is particularly preferably a photochromic compound represented by Formula (10) below. In Formula (10), R¹³, R²³, R³³, R⁴³, b3, and c3 are each the same as for Formula (9) above.

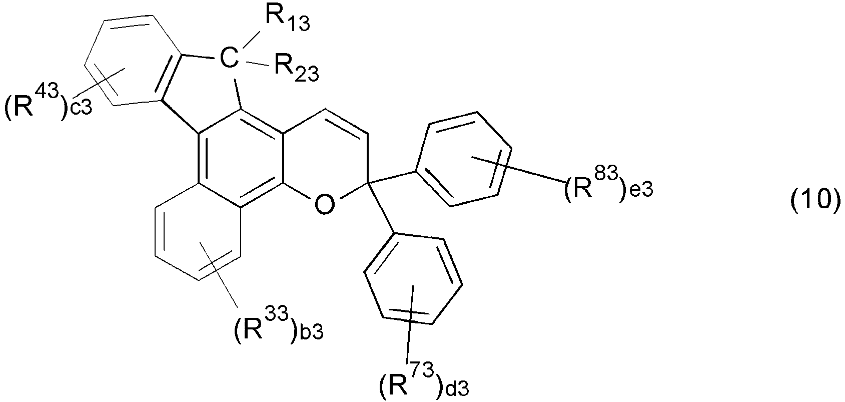

### (R⁷³ and R⁸³)

R⁷³ and R⁸³ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above. Suitable examples of R⁷³ and R⁸³ may be the same as those exemplified for R³ and R⁴.

d3 denotes a number of R⁷³ and is an integer of 0 or more and 5 or less. When d3 is 2 or more, R⁷³s may be the same as or different from each other.

When d3 is an integer of 2 or more and 5 or less and adjacent R⁷³s are present, two adjacent R⁷³s may be taken together with a carbon atom to which R⁷³s are attached to form a ring optionally including at least one atom selected from the group consisting of an oxygen atom, a sulfur atom, a carbon atom, and a nitrogen atom and the ring may have a substituent. Note that, the ring can also simultaneously have two or more atoms selected from the group consisting of an oxygen atom, a sulfur atom, a carbon atom, and a nitrogen atom.

e3 denotes a number of R⁸³ and is an integer of 0 or more and 5 or less. When e3 is 2 or more, R⁸³s may be the same as or different from each other.

When e3 is an integer of 2 or more and 5 or less and adjacent R⁸³s are present, two adjacent R⁸³s may be taken together with a carbon atom to which R⁸³s are attached to form a ring optionally including at least one atom selected from the group consisting of an oxygen atom, a sulfur atom, a carbon atom, and a nitrogen atom and the ring may have a substituent. Note that, the ring can also simultaneously have two or more atoms selected from the group consisting of an oxygen atom, a sulfur atom, a carbon atom, and a nitrogen atom.

For R⁷³ and R⁸³, each independently, two adjacent to each other may be taken together to form a ring group optionally including at least one selected from the group consisting of an oxygen atom, a sulfur atom, a carbon atom, and a nitrogen atom. This ring group is not particularly limited, and is preferably a ring having 5 to 8 atoms including a carbon atom to which R⁷³ and R⁸³ are attached. The ring may have a substituent. The substituent may be a substituent selected from a hydroxy group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Specific examples of these substituents are the same groups as described above. For the ring, it is preferred that a plurality of the rings be taken together to form the ring represented by Formula (X5) above.

Specific examples of the second photochromic compound C include the following compounds: in which Me denotes a methyl group and Pr denotes a propyl group.

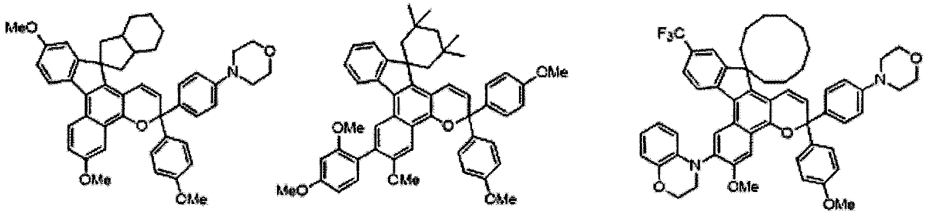

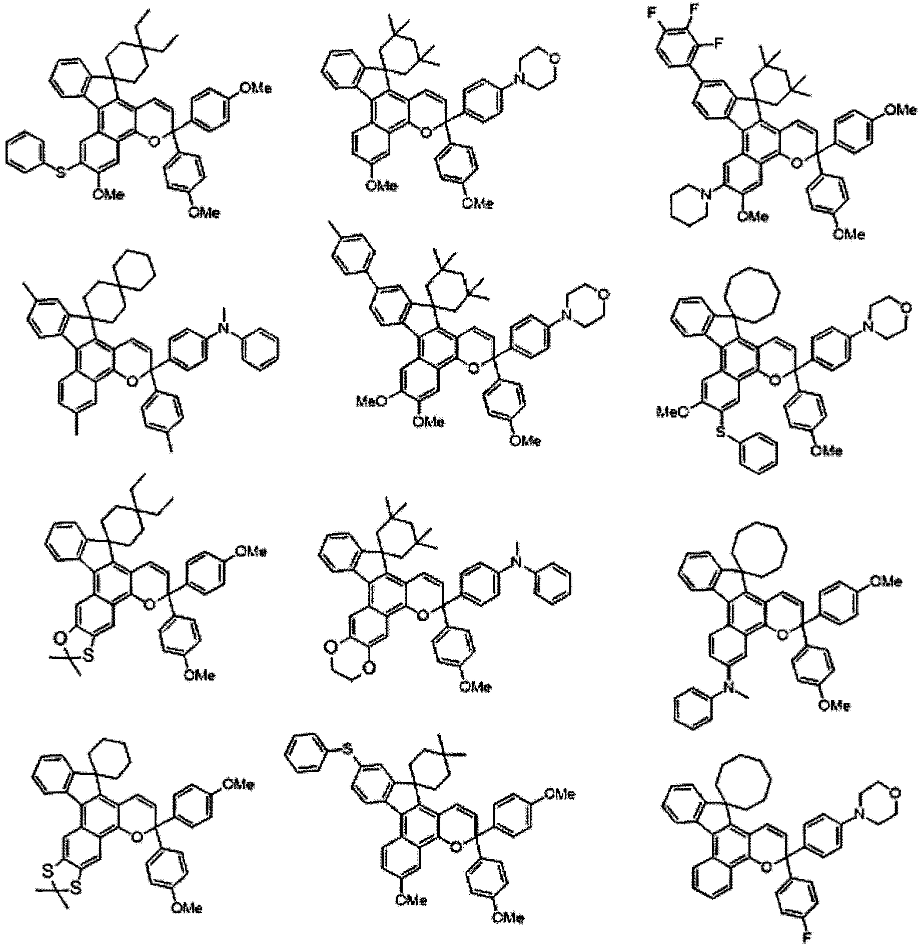

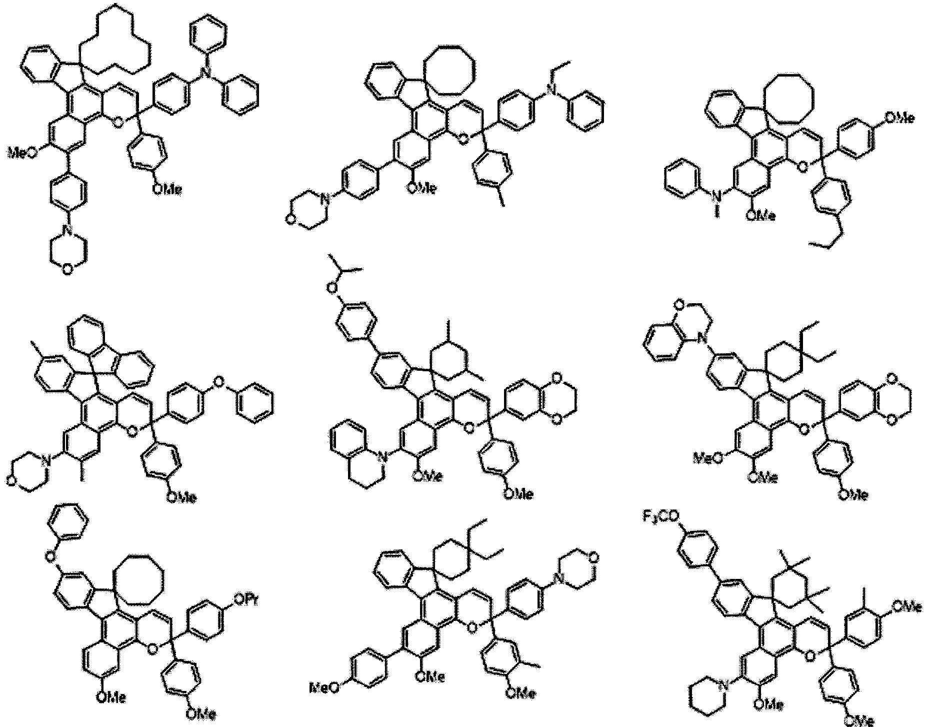

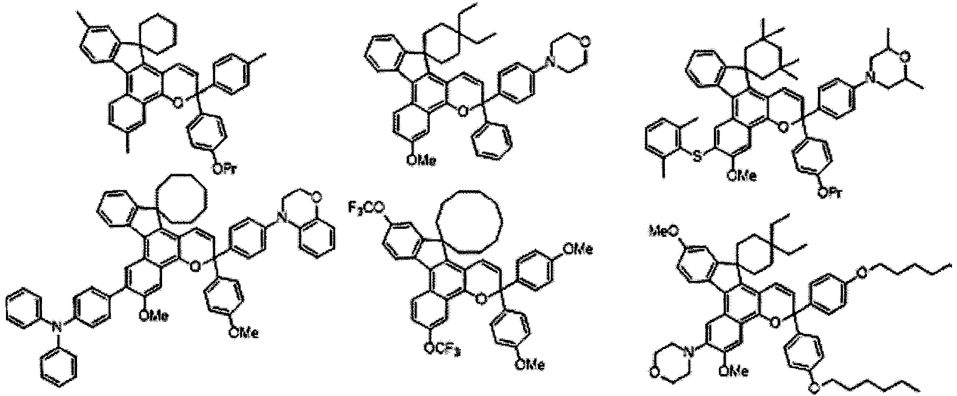

The second photochromic compound C can be synthesized by any of known methods.

### <Other photochromic compounds>

A photochromic composition of the present disclosure may include another photochromic compound in addition to a first photochromic compound and a second photochromic compound. The other photochromic compound includes a photochromic compound having a structure other than that of the first photochromic compound or the second photochromic compound. Examples of the other photochromic compound include a naphthopyran compound, a spirooxazine compound, a benzopyran compound, a spiropyran compound, a fulgide compound, or a diarylethene compound other than the first photochromic compound or the second photochromic compound.

A percentage of the other photochromic compound to the photochromic composition of the present disclosure depends on solubility in a matrix material, photochromicity, durability, etc., and it is preferably 20% by mass or less, more preferably 10% by mass or less, and further preferably 5% by mass or less. A lower limit of the percentage is 0% by mass. When the photochromic composition includes the other photochromic compound other than the first photochromic compound or the second photochromic compound, solubility in a matrix material or durability of a cured product may deteriorate. Therefore, the photochromic composition preferably consists of the first photochromic compound and the second photochromic compound.

### <Mixing ratio of photochromic compound>

For the photochromic composition, a percentage of the first photochromic compound to a total of the first photochromic compound and the second photochromic compound is preferably 5% by mass or more and 99% by mass or less. This percentage is more preferably 10% by mass or more and 90% by mass or less, further preferably 20% by mass or more and 80% by mass or less, particularly preferably 25% by mass or more and 75% by mass or less, and extremely preferably 30% by mass or more and 70% by mass or less.

The second photochromic compound may consist of a second photochromic compound A, or may consist of a second photochromic compound B, or may consist of a second photochromic compound C. The second photochromic compound may include only the second photochromic compound A and the second photochromic compound B, only the second photochromic compound A and the second photochromic compound C, or only the second photochromic compound B and the second photochromic compound C. The second photochromic composition may include all of the second photochromic compound A, the second photochromic compound B, and the second photochromic compound C.

The second photochromic compound preferably includes the second photochromic compound A from the viewpoint of enhancing long-term solubility. From the viewpoint of increasing a color developing density of a photochromic layer, the second photochromic compound B is preferably included. From the viewpoint of increasing a color fading rate of a photochromic layer, the second photochromic compound C is preferably included.

A percentage of a second photochromic compound A to a total of second photochromic compounds is preferably 0% by mass or more and 100% by mass or less, more preferably 10% by mass or more and 90% by mass or less, further preferably 15% by mass or more and 85% by mass or less, and particularly preferably 20% by mass or more and 80% by mass or less. From the viewpoint of enhancing long-term solubility, the percentage of a second photochromic compound A to a total of second photochromic compounds is preferably 20% by mass or more, more preferably 40% by mass or more, further preferably 60% by mass or more, particularly preferably 70% by mass or more, and extremely preferably 100%.

A percentage of a second photochromic compound B to a total of second photochromic compounds is preferably 0% by mass or more and 100% by mass or less, more preferably 10% by mass or more and 90% by mass or less, further preferably 15% by mass or more and 85% by mass or less, and particularly preferably 20% by mass or more and 80% by mass or less. From the viewpoint of increasing a color developing density of a photochromic layer, the percentage of a second photochromic compound B to a total of second photochromic compounds is preferably 30% by mass or more, more preferably 50% by mass or more, further preferably 60% by mass or more, particularly preferably 70% by mass or more, and extremely preferably 100%. From the viewpoint of increasing a color fading rate of a photochromic layer, the percentage of a second photochromic compound B to a total of second photochromic compounds is preferably 70% by mass or less, more preferably 50% by mass or less, and further preferably 30% by mass or less.

A percentage of a second photochromic compound C to a total of second photochromic compounds is preferably 0% by mass or more and 100% by mass or less, more preferably 10% by mass or more and 90% by mass or less, further preferably 15% by mass or more and 85% by mass or less, and particularly preferably 20% by mass or more and 80% by mass or less. From the viewpoint of increasing a color fading rate of a photochromic layer, the percentage of a second photochromic compound C to a total of second photochromic compounds is preferably 20% by mass or more, more preferably 30% by mass or more, further preferably 50% by mass or more, particularly preferably 70% by mass or more, and extremely preferably 100%.

A content of the first photochromic compound in a photochromic composition may be 1% by mass or more and 99% by mass or less.

A content of the first photochromic compound in a photochromic composition including the second photochromic compound B is 1% by mass or more and 99% by mass or less, and the content of the second photochromic compound B may be 1% by mass or more and 50% by mass or less.

A content of the first photochromic compound in a photochromic composition including the second photochromic compound C is 1% by mass or more and 99% by mass or less, and the content of the second photochromic compound C may be 1% by mass or more and 50% by mass or less.

A total content of the first photochromic compound, the second photochromic compound A, the second photochromic compound B, and the second photochromic compound C in a photochromic composition including the second photochromic compound A, the second photochromic compound B, and the second photochromic compound C may be 80% by mass or more and 100% by mass or less.

In a photochromic composition consisting of the first photochromic compound, the second photochromic compound A, the second photochromic compound B, and the second photochromic compound C, a content of the first photochromic compound may be 1% by mass or more and 99% by mass or less, a content of the second photochromic compound A may be 1% by mass or more and 99% by mass or less, a content of the second photochromic compound B may be 1% by mass or more and 99% by mass or less, and a content of the second photochromic compound C may be 10 by mass or more and 99% by mass or less. From the viewpoint of enhancing long-term solubility, a total content of the first photochromic compound and the second photochromic compound A in a photochromic composition is preferably 20% by mass or more, more preferably 40% by mass or more, further preferably 60% by mass or more, particularly preferably 70% by mass or more, and extremely preferably 80% by mass or more. From the viewpoint of enhancing long-term solubility and increasing a color fading rate of a photochromic layer, a photochromic composition consisting of the first photochromic compound, the second photochromic compound A, and the second photochromic compound C is preferred and a content of the second photochromic compound C relative to a total of the above-mentioned photochromic compounds is preferably 10% by mass or more and 60% by mass or less, more preferably 20% by mass or more and 50% by mass or less, and further preferably 25% by mass or more and 40% by mass or less. From the viewpoint of enhancing long-term solubility and increasing a color developing density of a photochromic layer, a photochromic composition consisting of the first photochromic compound, the second photochromic compound A, and the second photochromic compound B is preferred and a content of the second photochromic compound B relative to a total of the above-mentioned photochromic compounds is preferably 10% by mass or more and 60% by mass or less, more preferably 20% by mass or more and 50% by mass or less, and further preferably 25% by mass or more and 40% by mass or less.

A mixing ratio of the above-mentioned photochromic compounds can be analyzed, for example, as follows. First, a photochromic composition is purified by an isolation operation such as silica gel column chromatography, and then the thus-isolated photochromic compound is subjected to a proton nuclear magnetic resonance (¹H-NMR) analysis and a liquid chromatography-mass (LC-MS) analysis. Thus, for each photochromic compound, a structure can be identified, a content can be calculated, and a percentage can be determined.

Note that, if the photochromic composition is included in a cured product, it can be analyzed, for example, as follows. First, the cured product is impregnated with an organic solvent to extract the photochromic composition. The thus-extracted photochromic composition is purified by an isolation operation such as silica gel column chromatography to isolate each of two or more photochromic compounds. Each of the thus-isolated photochromic compounds can be subjected to a ¹H-NMR analysis and an LC-MS analysis to identify a structure thereof, calculate a content thereof, and determine a percentage thereof.

### <Other additives>

A photochromic composition of the present disclosure may include another additive as necessary. Examples of the additive include an organic solvent, a polymerizable monomer, etc.

The organic solvent is not particularly limited as long as a photochromic compound can dissolve thereinto. Examples of the organic solvent include acetone, methyl ethyl ketone, methyl isobutyl ketone, diethyl ketone, cyclohexanone, dioxane, toluene, N-methyl pyrrolidone, hexane, heptane, ethyl acetate, butyl acetate, dimethylformamide (DMF), dimethyl sulfoxide (DMSO), tetrahydrofuran (THF), etc.

The polymerizable monomer may be those described for the below-mentioned curable composition.

### <<Curable composition>>

A curable composition of the present disclosure includes a photochromic composition of the present disclosure; and at least one selected from the group consisting of a radical polymerizable monomer, a cationic polymerizable monomer, a compound having a polymerization reactive group, and a (thio)urethane(urea) polymer. Here, the (thio) urethane (urea) polymer includes at least one selected from the group consisting of a urethane polymer, a thiourethane polymer, a urethaneurea polymer, and a thiourethaneurea polymer.

The photochromic composition of the present disclosure is preferably used as a photochromic curable composition in combination with a polymerizable compound.

For the photochromic curable composition, the photochromic composition is preferably used in an amount of 0.001 parts by mass or more and 20 parts by mass or less relative to 100 parts by mass of the polymerizable compound, although it depends on a color developing intensity of a photochromic compound, a lens material selected, and a thickness of a lens. An amount of the photochromic composition is more preferably 1 part by mass or more and 10 parts by mass or less. The photochromic composition of the present disclosure can be incorporated at a relatively high concentration of 3 parts by mass or more due to its high solubility. An optimal amount to be incorporated depends on an application for which the photochromic curable composition is used. For example, a case where the photochromic curable composition is used as a thin-film optical article and a case where the photochromic curable composition is used as a thick-film optical article will be described below.

### (Use as thin film optical article)

For example, when a thin film of 10 µm or more and less than 1000 µm, e.g., about 100 µm (polymer film made by polymerization of the photochromic curable composition) is formed from the photochromic curable composition, 0.001 parts by mass or more and 20 parts by mass or less of a photochromic compound (or photochromic composition) relative to 100 parts by mass of other polymerizable monomers may be used to adjust a color tone.

### (Use as thick film optical article)

In the case of a thick cured product (polymeric molded product made by polymerization of the photochromic curable composition), e.g., a cured product having a thickness of 1 mm or more, 0.001 parts by mass or more and 5 parts by mass of a photochromic compound (or photochromic composition) relative to 100 parts by mass of the thick cured product or other polymerizable monomers that provide the thick cured product may be used to adjust a color tone.

### <Polymerizable compound>

As mentioned above, a photochromic composition is preferably used as a photochromic curable composition in combination with a polymerizable compound. Examples of the polymerizable compound may include a urethane- or urea-based polymerizable compound that can form a urethane bond or a urea bond, a compound having a polymerization reactive group, a radical polymerizable compound, an epoxy-based polymerizable compound, etc. The polymerizable compound is not particularly limited, and, for example, the polymerizable compound described in Patent Document 10 may be suitably used.

Among these, the below-mentioned polymerizable compounds are particularly suitably used.

### <Compound having polymerization reactive group>

An example of a compound having a polymerization reactive group includes a compound having an iso(thio)cyanate group (iso(thio)cyanate compound). The iso(thio)cyanate compound is a compound having an isocyanate group or an isothiocyanate group and may include both an isocyanate group and an isothiocyanate group. This compound is suitably used in combination with an active hydrogen-containing compound as described below. Examples of such an iso(thio)cyanate compound include, but are not limited to, the below-mentioned compounds.

### (Polyiso(thio)cyanate)

Polyiso(thio)cyanate is a compound having at least two or more iso(thio)cyanate groups per molecule. Examples of the polyiso(thio)cyanate include an aromatic polyiso(thio)cyanate having an aromatic ring such as m-xylene diisocyanate or 4,4'-diphenylmethane diisocyanate; an aliphatic polyiso(thio)cyanate such as norbornane diisocyanate or dicyclohexylmethane-4,4'-diisocyanate, etc.

### (Active hydrogen-containing compound)

An active hydrogen-containing compound is preferably a compound having a hydroxy group and/or a thiol group and particularly preferably a polyfunctional compound having two or more active hydrogens per molecule, but is not limited thereto. Specific examples of the active hydrogen-containing compound include a polyfunctional thiol compound such as pentaerythritol tetrakis(3-mercaptopropionate) or 4-mercaptomethyl-3,6-dithia-octanedithiol; a polyfunctional alcohol such as trimethylolpropane or pentaerythritol, etc.

### (Radical polymerizable compound)

A radical polymerizable compound includes a polyfunctional radical polymerizable compound and a monofunctional radical polymerizable compound. Each of these can be used alone, or a plurality of them can be used in combination. Examples of a radical polymerizable substituent include a group having an unsaturated double bond, i.e., a vinyl group including a styryl group, a (meth)acryl group, or an allyl group, etc.

The polyfunctional radical polymerizable compound is a compound having two or more radical polymerizable substituents per molecule. This polyfunctional radical polymerizable compound includes a first polyfunctional radical polymerizable compound having 2 to 10 radical polymerizable substituents and a second polyfunctional radical polymerizable compound having more than 10 radical polymerizable substituents.

The first polyfunctional radical polymerizable compound is not particularly limited, and more preferably has 2 to 6 radical polymerizable substituents. Specific examples thereof are as follows.

### (Polyfunctional (meth)acrylic acid ester compound)

An polyfunctional (meth)acrylic acid ester compound such as ethylene glycol di(meth)acrylate, diethylene glycol di(meth)acrylate, triethylene glycol di(meth)acrylate, tetraethylene glycol di(meth)acrylate, polyethylene glycol di(meth)acrylate, polypropylene glycol di(meth)acrylate, polytetramethylene glycol di(meth)acrylate, polyalkylene glycol di(meth)acrylate, di(meth)acrylate composed of a random or block structure of a plurality of polyalkylene glycols, ethylene glycol bisglycidyl(meth)acrylate, neopentyl glycol di(meth)acrylate, bisphenol A di(meth)acrylate, 2,2-bis(4-(meth)acryloyloxyethoxyphenyl)propane, 2,2-bis(3,5-dibromo-4-(meth)acryloyl, oxyethoxyphenyl)propane, trimethylolpropane tri(meth)acrylate, tetramethylolmethane tri(meth)acrylate, tetramethylolmethane tetra(meth)acrylate, trimethylolpropane triethylene glycol tri(meth)acrylate, di(trimethylolpropane) tetra(meth)acrylate, glycerol tri(meth)acrylate, etc.; a compound in which an end of a polyalkylene compound is modified with a (meth)acrylate group; a compound in which an end of a polycarbonate compound is modified with a (meth)acrylate group; a compound in which an end of a polyester compound is modified with a (meth)acrylate group; a polyfunctional (meth)acrylate compound having a urethane bond, such as a reaction product of a polyisocyanate compound having two or more isocyanate groups within its molecule, a polyol compound having two or more hydroxy groups within its molecule, and a hydroxy group-containing (meth)acrylate.

### (Polyfunctional allyl compound)

Diallyl phthalate, diallyl terephthalate, diallyl isophthalate, diallyl tartrate, diallyl epoxysuccinate, diallyl fumarate, diallyl chlorendate, diallyl hexaphthalate, diallyl carbonate, allyl diglycol carbonate, trimethylolpropane triallyl carbonate.

### (Polyfunctional thio(meth)acrylic acid ester compound)

1,2-bis(methacryloylthio)ethane, bis(2-acryloylthioethyl) ether, 1,4-bis(methacryloylthiomethyl)benzene.

### (Radical polymerizable compound)

Examples of the second polyfunctional radical polymerizable compound having more than 10 radical polymerizable substituents include a compound having a relatively large molecular weight such as a silsesquioxane compound having a radical polymerizable substituent or a polyrotaxane compound having a radical polymerizable substituent.

The monofunctional radical polymerizable compound is a compound having one radical polymerizable substituent in a molecule, and specific examples thereof include, but are not limited to, the below-mentioned compounds.

### (Unsaturated carboxylic acid)

Acrylic acid, methacrylic acid, maleic anhydride.

### ((Meth)acrylic acid ester)

Methyl (meth)acrylate, benzyl methacrylate, phenyl methacrylate.

### (Monofunctional (meth)acrylic acid ester compound)

2-Hydroxyethyl methacrylate, glycidyl (meth)acrylate, β-methyl glycidyl (meth)acrylate, bisphenol A-monoglycidyl ether methacrylate, 4-glycidyloxy methacrylate, 3-(glycidyl-2-oxyethoxy)-2-hydroxy, propyl methacrylate, 3-(glycidyloxy-1-isopropyloxy)-2-hydroxypropyl acrylate, 3-glycidyloxy-2-hydroxypropyloxy)-2-hydroxypropyl acrylate.

### (Fumaric acid ester)

Diethyl fumarate, diphenyl fumarate.

### (Thio(meth)acrylic acid)

Methyl thioacrylate, benzyl thioacrylate, benzyl thiomethacrylate.

### (Vinyl Compound)

Divinylbenzene, styrene, chlorostyrene, tylstyrene, vinylnaphthalene, α-methylstyrene dimer, bromostyrene.

The radical polymerizable compound may be used alone, or a mixture of a plurality of them may also be used. In this case, preferably 80 to 100 parts by mass of the polyfunctional radical polymerizable compound and 0 to 20 parts by mass of the monofunctional radical polymerizable compound, and more preferably 90 to 100 parts by mass of the polyfunctional radical polymerizable compound and 0 to 10 parts by mass of the monofunctional radical polymerizable compound are used per 100 parts by mass of a total of the radical polymerizable compounds. Furthermore, preferably 80 to 100 parts by mass of the first polyfunctional radical polymerizable compound, 0 to 20 parts by mass of the second polyfunctional radical polymerizable compound, and 0 to 20 parts by mass of the monofunctional radical polymerizable compound, and further preferably 85 to 100 parts by mass of the first polyfunctional radical polymerizable compound, 0 to 10 parts by mass of the second polyfunctional radical polymerizable compound, and 0 to 10 parts by mass of the monofunctional radical polymerizable compound are used per 100 parts by mass of a total of the radical polymerizable compounds.

### (Epoxy polymerizable compound)

An epoxy polymerizable compound can be broadly classified into an aliphatic epoxy compound, an alicyclic epoxy compound, and an aromatic epoxy compound. Each of these can be used alone, or a plurality of them can be used in combination.

### (Aliphatic epoxy compound)

Examples thereof include ethylene oxide, 2-ethyloxirane, butyl glycidyl ether, phenyl glycidyl ether, 2,2'-methylenebisoxirane, 1,6- hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, diglycidyl ether of tris(2-hydroxyethyl)isocyanurate, or triglycidyl ether of tris(2-hydroxyethyl)isocyanurate.

### (Alicyclic epoxy compound)

Isophoronediol diglycidyl ether or bis-2,2-hydroxycyclohexylpropane diglycidyl ether is exemplified.

### (Aromatic epoxy compounds)

Resorcin diglycidyl ether, bisphenol A diglycidyl ether, bisphenol F diglycidyl ether, bisphenol S diglycidyl ether, orthophthalic acid diglycidyl ester, phenolic novolac polyglycidyl ether, or cresol novolac polyglycidyl ether is exemplified.

In addition to the above-mentioned compounds, an epoxy-based polymerizable compound having a sulfur atom along with an epoxy group in a molecule can also be used. Such a sulfur atom-containing epoxy-based polymerizable compound contributes especially to an increase in a refractive index and includes a chain aliphatic compound and a cyclic aliphatic compound. Specific examples thereof are as follows.

### (Chain aliphatic sulfur atom-containing epoxy-based polymerizable compound)

Bis(2,3-epoxypropyl)sulfide, bis(2,3-epoxypropyl)disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis (2,3-epoxypropylthiomethyl)propane, 2,2-bis(2,3-epoxypropylthiomethyl)-1-(2,3-epoxypropylthio)butane.

### (Cyclic aliphatic sulfur-atom containing epoxy-based polymerizable compound)

1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, 2,5-bis(2,3-epoxypropylthiomethyl)-2,5-dimethyl-1,4-dithiane.

### (Various compounding agents)

Various known compounding agents may be incorporated into a curable composition providing that the effect is not impaired. Examples of the compounding agent include, for example, various stabilizing agents such as a releasing agent, a UV absorbing agent, an IR absorbing agent, a UV stabilizing agent, an antioxidizing agent, an anti-coloring agent, an antistatic agent, a fluorescent dye, a dye, a pigment, a perfume, etc. A solvent or a leveling agent can also be incorporated. A thiol such as t-dodecyl mercaptan can be incorporated as a polymerization-regulating agent.

Among the above-mentioned compounding agents, the UV stabilizing agent is suitably used from the viewpoint of improving durability of a photochromic compound. A hindered amine light stabilizing agent, a hindered phenol antioxidizing agent, a sulfur antioxidizing agent, etc., are known as such a UV stabilizing agent. A particularly suitable UV stabilizing agent is as follows:

Bis(1,2,2,6,6-pentamethyl-4-piperidyl)sebacate; ADK STAB LA-52, LA-57, LA-62, LA-63, LA-67, LA-77, LA-82, LA-87 (manufactured by ADEKA CORPORATION); 2,6-di-tert-butyl-4-methyl-phenol, ethylene bis(oxyethylene) bis[3-(5-tert-butyl-4-hydroxy-m-tolyl)propionate]; IRGANOX 1010, 1035, 1075, 1098, 1135, 1141, 1222, 1330, 1425, 1520, 259, 3114, 3790, 5057, 565 (manufactured by BASF). An amount of such a UV stabilizing agent to be used is not particularly limited providing that the effect is not impaired, and is usually in a range of 0.001 to 10 parts by mass, particularly 0.01 to 1 part by mass per 100 parts by mass of a photochromic curable composition.

A UV-absorbing agent may also be used in addition to the UV stabilizing agent. Known UV-absorbing agents such as a benzophenone compound, a benzotriazole compound, a cyanoacrylate compound, a triazine compound, or a benzoate compound can be used as the UV-absorbing agent, with a cyanoacrylate compound or a benzophenone compound being particularly preferred. The above-mentioned UV stabilizing agent is preferably used in a range of 0.001 to 5 parts by mass relative to 100 parts by mass of a photochromic curable composition including a photochromic composition and a polymerizable compound.

### <Method for using photochromic curable composition; optical article>

A photochromic curable composition is cured to obtain a photochromic cured product. Polymerization curing for producing the photochromic cured product is performed by radical polymerization, ring-opening polymerization, anionic polymerization, or condensation polymerization using irradiation with an active energy ray such as an ultraviolet ray, an α-ray, a β-ray, or a γ-ray; heat; or a combination thereof. In other words, an appropriate polymerization method may be employed depending on a type of a polymerizable compound or a polymerization accelerator and a form of a photochromic cured product to be formed.

When a curable composition containing a polymerizable compound is thermally polymerized, a temperature affects a property of the resulting photochromic cured product.

Such a temperature condition cannot be sweepingly limited since it is affected by a type or amount of a thermal polymerization initiator or a type of polymerizable compound. However, in general, a method in which polymerization is started at a relatively low temperature, followed by heating slowly is suitable. Polymerization time also varies depending on various factors, as is the case with temperature. Therefore, although it is suitable to determine an optimum time in advance in accordance with these conditions, in general, the conditions are preferably selected so that polymerization is completed in 2 to 48 hours. In the case of obtaining a photochromic laminated sheet, polymerization is performed at a temperature at which a reaction between polymerizable functional groups proceeds, and optimal temperature and time are preferably determined to achieve a desired molecular weight.

When a curable composition is photo-polymerized, among the polymerization conditions, especially UV intensity affects a property of the resulting photochromic cured product. Such an irradiation condition cannot be sweepingly limited since it is affected by a type or amount of a photoinitiator or a type of a polymerizable monomer. However, in general, the conditions are preferably selected so as to give photoirradiation with 50 to 500 mW/cm² of UV light at a wavelength of 365 nm for 0.5 to 5 minutes.

### [Optical article]

A photochromic composition of the present disclosure can be widely used as a photochromic material, and can be used, for example, as various memory materials alternative to a silver halide photosensitive material, a copying material, a photosensitive material for printing, a memory material for a cathode ray tube, a photosensitive material for a laser, a photosensitive material for holography, and various other memory materials. The photochromic material can also be used as a photochromic lens material, an optical filter material, a display material, or a material for a light meter or decoration.

The photochromic composition of the present disclosure is particularly suitable for a photochromic lens application. A photochromic lens is suitable as a lens for eyeglasses such as sunglasses. Any known method for producing a photochromic lens can be employed, as long as the method provides uniform light control performance.

In the case where a photochromic property is developed by a kneading method, a photochromic cured product in a form of an optical material such as a lens may be obtained by injecting the above-mentioned curable composition between glass molds held with an elastomeric gasket or a spacer and then heating the curable composition in an air furnace or cast-polymerizing the curable composition by irradiation with an active energy ray such as ultraviolet ray depending on a type of a polymerizable compound or a polymerization accelerator.

In the case where a photochromic property is developed by a lamination method, a photochromic layer made of a photochromic cured product is formed on a surface of an optical substrate by dissolving a curable composition in an organic solvent as appropriate to prepare a coating solution; coating a surface of an optical substrate such as a lens substrate with the coating solution by spin coating, dipping, etc.; drying to remove the organic solvent; and then polymerization-curing the curable composition with UV irradiation, heating, etc., in an inert gas such as nitrogen (coating method).

Furthermore, a photochromic layer made of a photochromic cured product can also be formed on a surface of an optical substrate by casting polymerization using an inner mold in which an optical substrate such as a lens substrate is placed face-to-face with a glass mold so that a predetermined void is formed, the curable composition is injected into the void, and the curable composition is polymerization-cured in this state by UV irradiation, heating, etc. (casting polymerization method).

In the case where a photochromic layer is formed on a surface of an optical substrate by the lamination method (coating method and casting polymerization method) as described above, the surface of the optical substrate may be chemically treated with an alkali solution, an acid solution, etc., or physically treated with corona discharge, plasma discharge, polishing, etc., in advance in order to enhance adhesion between the photochromic layer and the optical substrate. Of course, it is also possible to provide a transparent adhesive resin layer on the surface of the optical substrate.

In addition, when a photochromic property is developed by a binder method, a photochromic sheet is made by sheet-molding using a curable composition, sandwiched between two transparent sheets (optical sheets), and polymerization-cured as described above to obtain a photochromic laminate with a photochromic layer as an adhesive layer.

In this case, coating with a coating solution in which a curable composition is dissolved in an organic solvent can also be employed to produce the photochromic sheet.

The thus-produced photochromic laminate is, for example, mounted in a mold and then a thermoplastic resin (e.g., polycarbonate) for an optical substrate such as a lens is injection-molded thereon to obtain an optical substrate such as a lens with a photochromic property in a predetermined shape.

The photochromic laminate can also be adhered to a surface of an optical substrate with, for example, an adhesive to obtain a photochromic lens.

Note that, when a photochromic laminate is produced as described above, it is preferred to use, as a polymerizable compound, a urethane- or urea-based polymerizable compound, especially a urethane-based polymerizable compound to adjust so as to form polyurethane from the viewpoint of high adhesion especially to an optical substrate.

The above-mentioned curable composition can develop a photochromic property with an excellent color developing density at a high temperature.

A photochromic layer or a photochromic cured product formed from a curable composition can be subjected to post-processing such as staining using a dye such as a disperse dye; formation of a hard coat film using a silane coupling agent or a hard coat agent containing as a main component a sol of silicon, zirconium, antimony, aluminum, tin, tungsten, etc.; formation of a thin film by vapor deposition of a metal oxide such as SiO₂, TiO₂, ZrO₂, etc.; antireflection treatment by a thin film coated with an organic polymer; antistatic treatment; anti-abrasion treatment, etc., depending on its application.

### EXAMPLES

The present invention will be described in detail with reference to Examples and Comparative Examples, but the present invention is not limited to Examples.

### <<Examples 1 to 13 and Comparative Examples 1 to 6>>

First, the below-mentioned photochromic compounds were prepared. Each of the photochromic compounds shown in Tables 1 to 3 was mixed with the below-mentioned radical polymerizable composition to obtain a photochromic curable composition.

In Table 1, an incorporated amount (part(s) by mass) of each photochromic compound (chromene compound) when a total mass of radical polymerizable monomers (total mass of a polymerizable composition) is taken as 100 parts by mass is represented by a numerical value in parentheses.

### <Radical polymerizable composition>

### (Polymerizable compound)

Polyethylene glycol dimethacrylate (average molecular weight: 736): 50 parts by mass
Polycarbonediol diacrylate (average molecular weight: 620) obtained by phosgenation of pentamethylene glycol and hexamethylene glycol: 22 parts by mass
Trimethylolpropane trimethacrylate: 10 parts by mass
Dipentaerythritol tetramethacrylate: 15 parts by mass
γ-Methacryloyloxypropyltrimethoxysilane: 2 parts by mass
Glycidyl methacrylate: 1 part by mass.

### (Other Components)

Phenylbis(2,4,6-trimethylbenzoyl)phosphine oxide: 0.3 parts by mass
Ethylene bis(oxyethylene) bis[3-(5-tert-butyl-4-hydroxy-m-tolyl) propionate]: 1 part by mass
Bis(1,2,2,6,6-pentamethyl-4-piperidyl)sebacate: 3 parts by mass

### <Photochromic compound>

### (First photochromic compound or second photochromic compound A)

### PC1-1

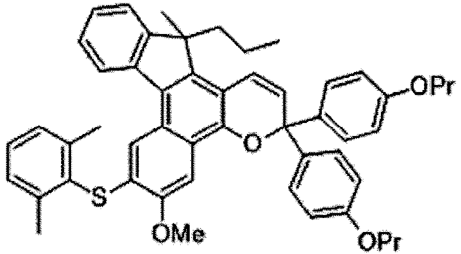

### PC1-2

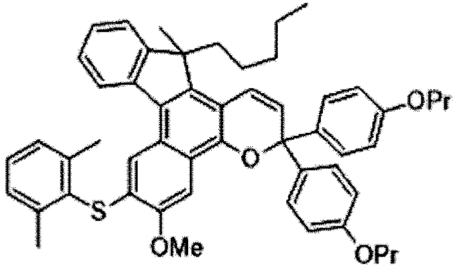

### PC1-3

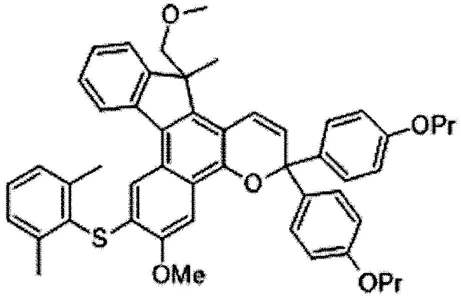

### PC1-4

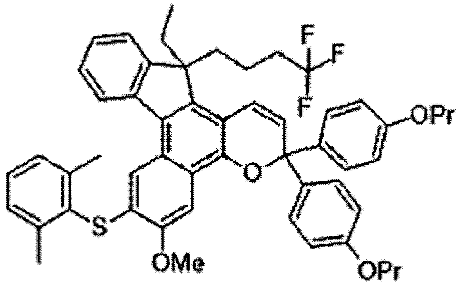

### PC1-5

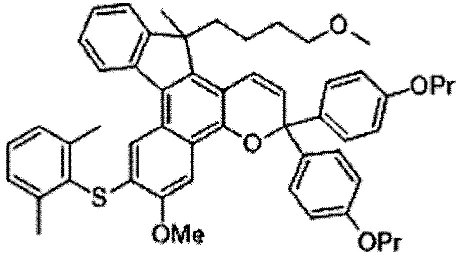

### PC1-6

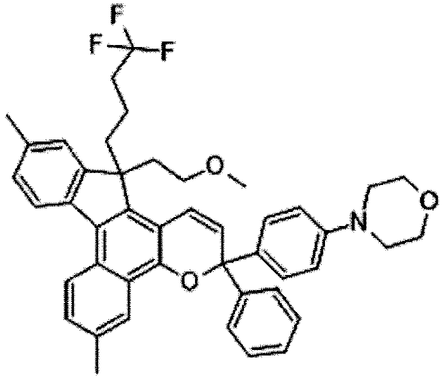

### PC1-7

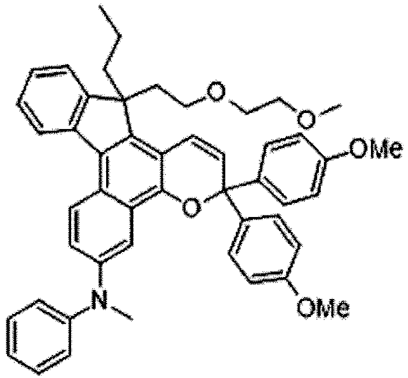

### PC1-8

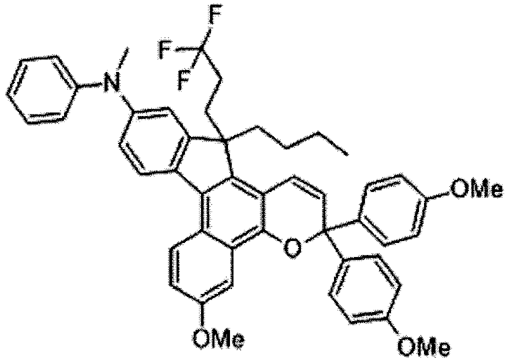

### PC1-9

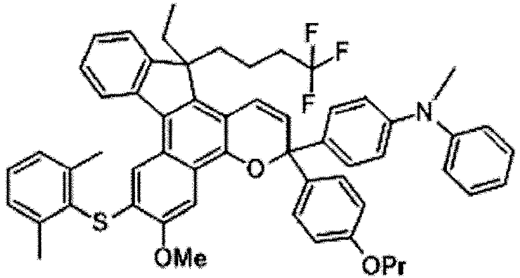

### (Second photochromic compound B)

### PC2B-1

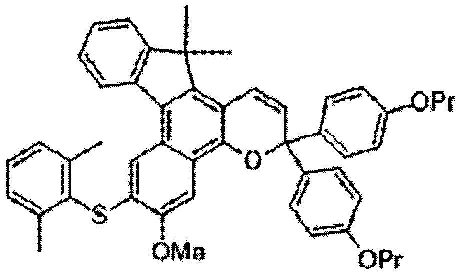

### PC2B-2

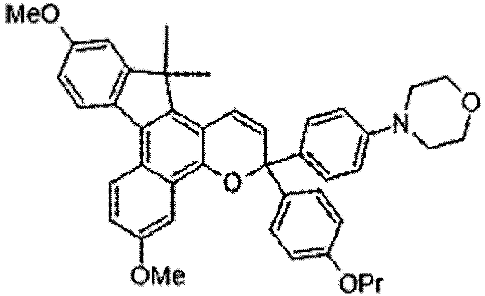

### PC2B-3

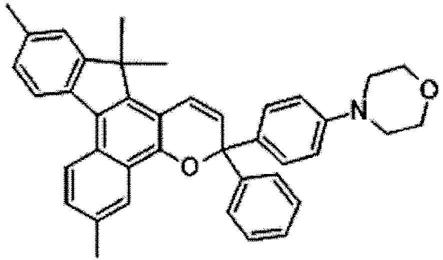

### (Second photochromic compound C)

### PC2C-1

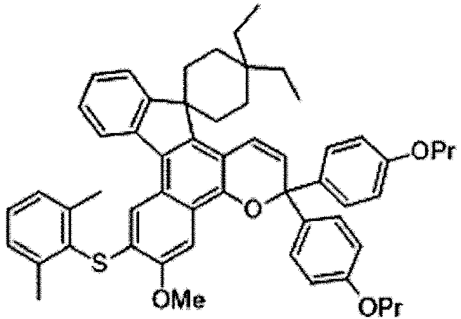

### PC2C-2

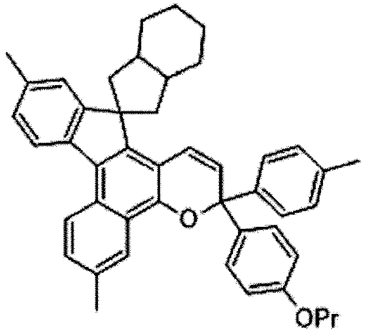

### PC2C-3

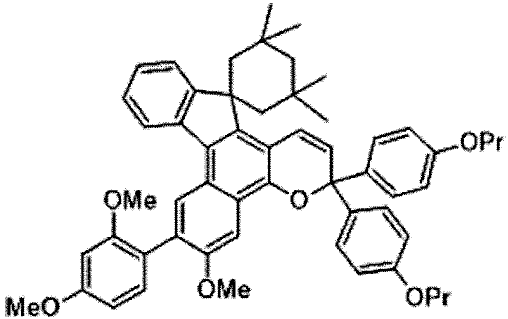

### PC2C-4

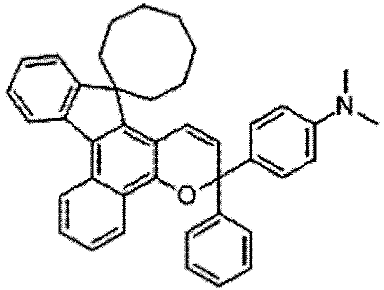

### (Other photochromic compounds)

### PC2D-1

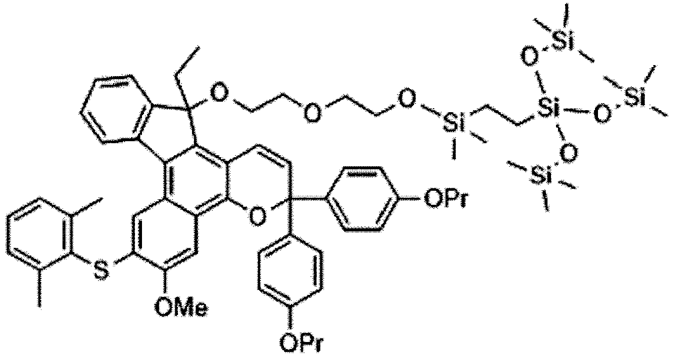

### PC2D-2

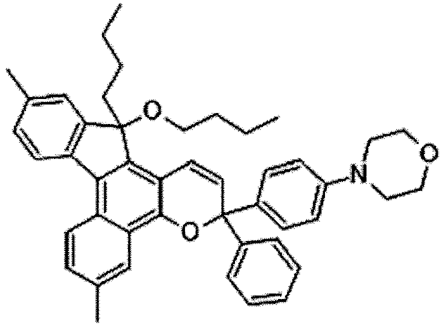

### <Synthesis Method>

### [Synthesis of PC1-1]

### (Step 1)

First, 200 mL of toluene was added to 12.1 g (20.0 mmol) of a compound represented by Formula (1-1-1) below, which was synthesized with reference to the method described in Patent Document 11, and heated to distill 50 mL of the toluene off under atmospheric pressure. After the toluene was distilled off, the resultant was cooled until an internal temperature was -20°C, and 15 mL of n-BuLi (1.6 M hexane solution) was added dropwise thereto while maintaining -15°C or less. After consumption of the raw materials was confirmed, 2.6 g (30.2 mmol) of methyl propyl ketone was added dropwise thereto while maintaining -15°C or less. After stirring for 1 hour, the resultant was slowly heated to room temperature. Then, the resultant was partitioned by adding 150 mL of water thereto. Washing with water was repeated until a pH of an aqueous layer was 7, and the resulting organic layer was concentrated, followed by purification by chromatography on silica gel to obtain a compound represented by Formula (1-1-2) below at a yield of 83%.

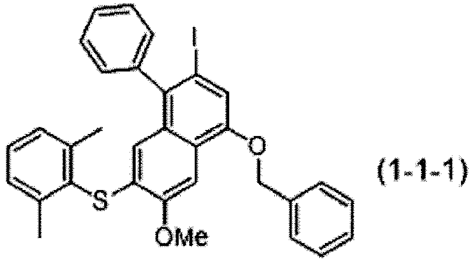

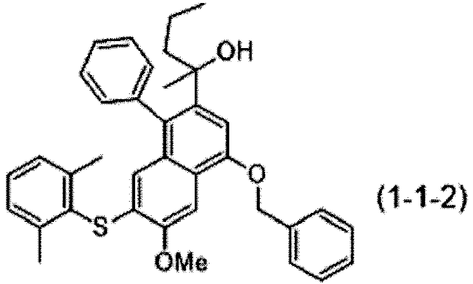

### (Step 2)

Then, 9.34 g (16.6 mmol) of the compound represented by Formula (1-1-2) was dissolved in 75 mL of tetrahydrofuran, 1.4 g of 5% Pd/C (50% water content) was added thereto, followed by reacting under pressure with hydrogen gas at 0.05 to 0.1 MPa. After consumption of the raw materials was confirmed, the Pd/C was filtered off and a solvent was removed from the resulting organic layer to obtain a compound represented by Formula (1-1-3) below at a yield of 100%.

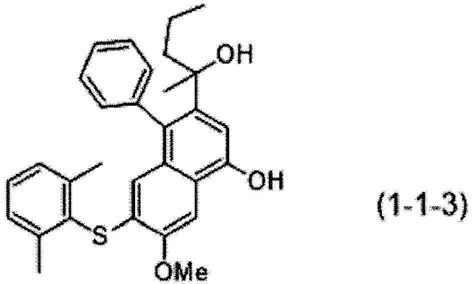

### (Step 3)

One hundred milliliters of toluene was added to 5.0 g (26.1 mmol) of p-toluenesulfonic acid monohydrate, and azeotropic dehydration was performed until a water content in the toluene was 109 ppm. After the azeotropic dehydration, 7.85 g of the compound represented by Formula (1-1-3) was added thereto and heated at 100°C. After consumption of the raw materials, the resultant was cooled to room temperature and partitioned by adding 48 g of a 5% sodium hydrogen carbonate aqueous solution thereto and stirring. The resultant was partitioned by adding 100 mL of water thereto. Washing with water was repeated until a pH of an aqueous layer was 7, and the resulting organic layer was concentrated, followed by purification by chromatography on silica gel to obtain a compound represented by Formula (1-1-4) below at a yield of 87%.

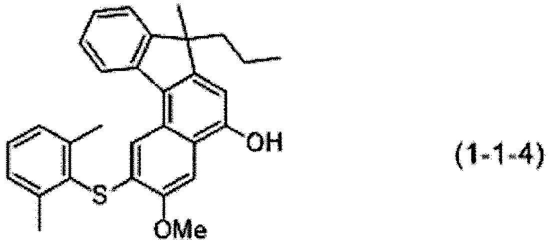

### (Step 4)

First, 4.55 g (10.0 mmol) of the compound represented by Formula (1-1-4) and 3.89 g (12.0 mmol) of propargyl alcohol represented by Formula (1-5) below were dissolved in 80 mL of toluene, and 0.25 g (1.0 mmol) of pyridinium p-toluenesulfonate was added thereto and stirred at 85°C for 1 hour. After the compound represented by Formula (1-1-4) was consumed, the resultant was cooled to room temperature and partitioned by adding 80 mL of water thereto. A solvent was removed from the resulting organic layer, followed by purification by chromatography on silica gel and recrystallization with a mixed solvent of ethanol/acetonitrile to obtain PC-1-1 at a yield of 76%.

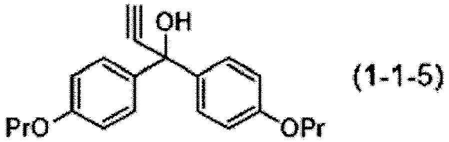

### [Synthesis of PC1-2]

PC1-2 was synthesized in the same manner as the synthesis of PC1-1, except that 2-heptanone was used instead of methyl propyl ketone in the first step.

### [Synthesis of PC1-3]

PC1-3 was synthesized in the same manner as the synthesis of PC1-1, except that methoxyacetone was used instead of methyl propyl ketone in the first step.

### [Synthesis of PC1-4]

PC1-4 was synthesized in the same manner as the synthesis of PC1-1, except that 1,1,1-trifluoro-2-hexanone was used instead of methyl propyl ketone in the first step.

### [Synthesis of PC1-5]

PC1-5 was synthesized in the same manner as the synthesis of PC1-1, except that 6-methoxy-2-hexanone was used instead of methyl propyl ketone in the first step.

### [Synthesis of PC1-6]

### (Step 1)

A compound represented by Formula (1-6-2) below was synthesized in the same manner as PC1-1, except that a compound represented by Formula (1-6-1) below, which was synthesized from 4,4'-dimethylbenzophenone with reference to the method described in Patent Document 12, was used instead of the compound represented by Formula (1-2-1) and 6,6,6-trifluoro-1-methoxy-3-hexanone was used instead of methyl propyl ketone in the first step.

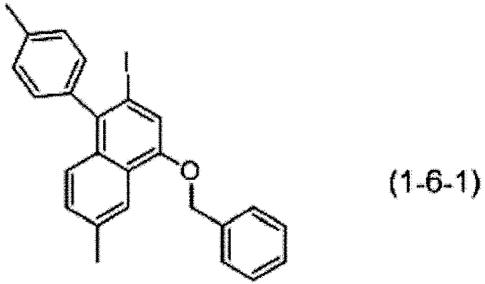

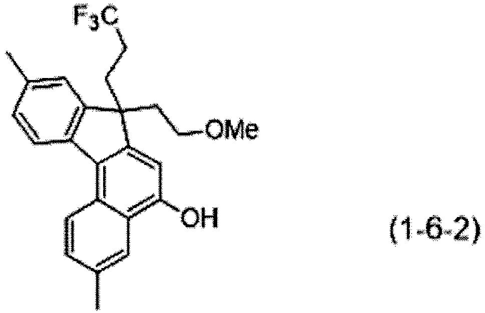

### (Step 2)

PC1-6 was synthesized in the same manner as PC1-1, except that the compound represented by Formula (1-6-2) was used instead of the compound represented by Formula (1-1-4) and the compound represented by Formula (1-6-3) below was used instead of the compound represented by Formula (1-1-5) in the fourth step.

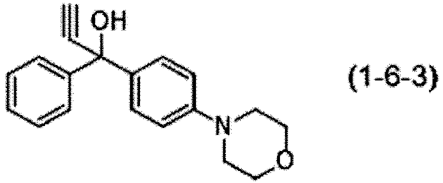

### [Synthesis of PC1-7]

### (Step 1)

First, 24.3 g (56.2 mmol) of a compound represented by Formula (1-7-1) below, which was synthesized from 4-bromobenzophenone with reference to the method described in Patent Document 12, 550 mL of toluene, 8.4 g (78.6 mmol) of N-methylaniline, and 21.6 g (224.6 mmol) of tertiary-butoxy sodium were added and stirred under reduced pressure to remove dissolved oxygen. Then, 0.52 g (0.54 mmol) of Pd₂(dba)₃ and 1.01 g (2.3 mmol) of X-phos were added to the resulting reaction liquid and heated to 80°C.

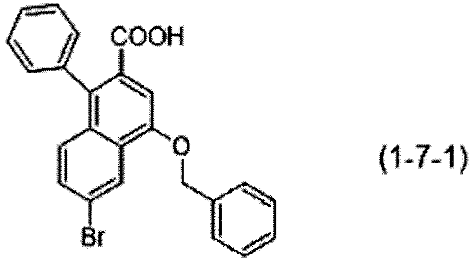

Heating was continued until the raw materials were exhausted. After the reaction was completed, the resultant was cooled to room temperature and filtered. Then, 500 mL of tetrahydrofuran and 10% hydrochloric acid were added to the resulting filtrate for neutralization, and then the resultant was partitioned. The resulting organic layer was concentrated and then purified by reslurrying with 150 mL of methanol to obtain a carboxylic acid compound represented by Formula (1-7-2) below at a yield of 90%.

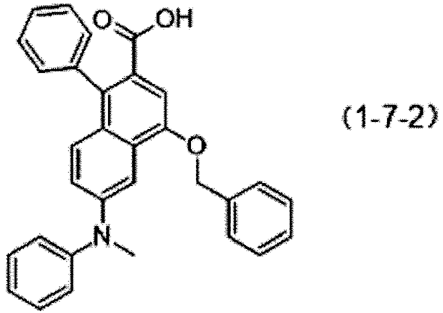

### (Step 2)

A compound represented by Formula (1-7-3) below was synthesized with reference to the method described in Patent Document 12, except that the carboxylic acid compound obtained in the first step was used.

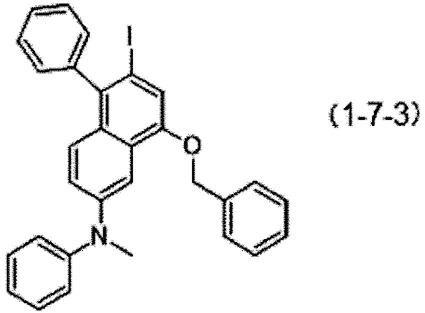

### (Step 3)

A compound represented by Formula (1-7-4) below was synthesized in the same manner as PC1-1, except that the compound represented by Formula (1-7-3) was used instead of the compound represented by Formula (1-2-1) and 1-(2-methoxyethoxy)-3-hexanone was used instead of methyl propyl ketone in the first step.

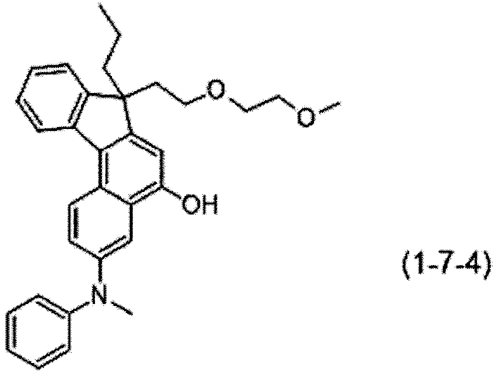

### (Step 4)

PC1-7 was synthesized in the same manner as PC1-1, except that the compound represented by Formula (1-7-4) was used instead of the compound represented by Formula (1-1-4) and a compound represented by Formula (1-7-5) below was used instead of the compound represented by Formula (1-1-5) in the fourth step.

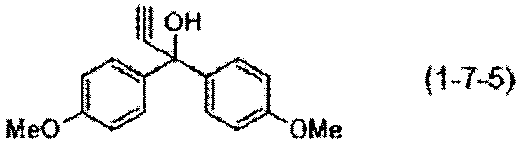

### [Synthesis of PC1-8]

### (Step 1)

A compound represented by Formula (1-8-1) below was synthesized in the same manner as PC1-7, except that 4-bromo-4'-methoxybenzophenone was used instead of 4-bromophenone in the first step.

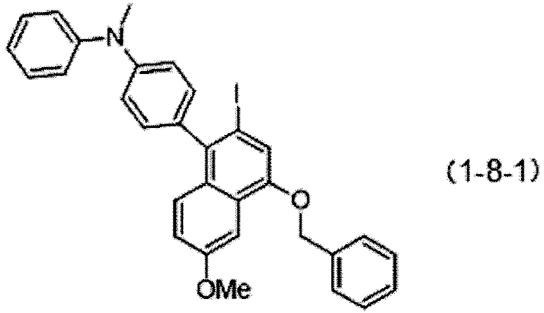

### (Step 2)

A compound represented by Formula (1-8-2) below was synthesized in the same manner as PC1-1, except that the compound represented by Formula (1-8-1) was used instead of the compound represented by Formula (1-2-1) above and 1,1,1-trifluoro-4-octanone was used instead of methyl propyl ketone in the first step.

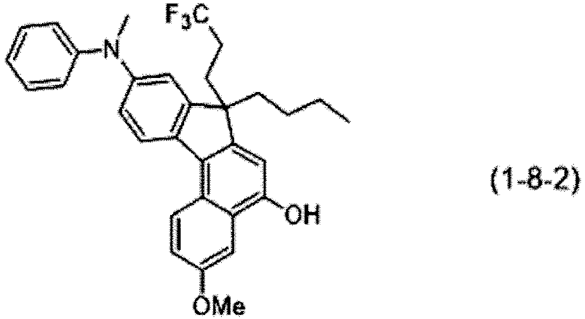

### (Step 3)

PC1-8 was synthesized in the same manner as PC1-7, except that the compound represented by Formula (1-8-2) was used instead of the compound represented by Formula (1-7-4) in the fourth step.

### [Synthesis of PC1-9]

PC1-9 was synthesized in the same manner as PC1-4, except that a compound represented by Formula (1-9-1) below was used instead of the compound represented by Formula (1-1-5).

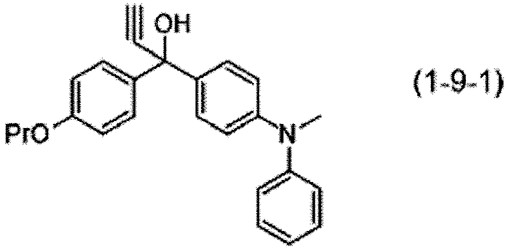

### [Synthesis of PC2B-1]

PC2B-1 was synthesized in the same manner as the synthesis of PC1-1, except that acetone was used instead of methyl propyl ketone in the first step.

### [Synthesis of PC2B-3]

PC2B-3 was synthesized in the same manner as the synthesis of PC1-6, except that acetone was used instead of 6,6,6-trifluoro-1-methoxy-3-hexanone in the first step.

### [Synthesis of PC2D-1]

PC2D-1 was synthesized by reacting a compound represented by Formula (2D-1-1) below, which was synthesized with reference to the method described in Patent Document 13, with tris(trimethylsiloxy)silyl ethyl chlorosilane with reference to the method described in Patent Document 3.

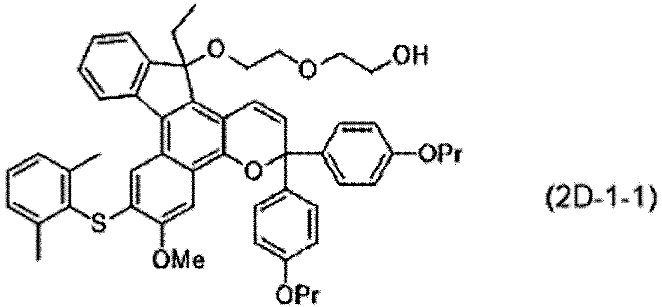

**[Table 1]**

| | First photochromic compound | Second photochromic compound A | Second photochromic compound B | Second photochromic compound C | Other photochromic compounds |
|---|---|---|---|---|---|
| Example 1 | PC1-1(4.0) | PC1-3(1.0) | - | - | - |
| Example 2 | PC1-5(3.0) | PC1-3(2.0) | - | - | - |
| Example 3 | PC1-5(3.0) | PC1-3(1.0) | - | - | - |
| | | PC1-2(1.0) | | | |
| Example 4 | PC1-1(4.0) | - | PC2B-1(1.0) | - | - |
| Comparative Example 1 | PC1-1 (5.0) | - | - | - | - |

**[Table 2]**

| | First photochromic compound | Second photochromic compound A | Second photochromic compound B | Second photochromic compound C | Other photochromic compounds |
|---|---|---|---|---|---|
| Example 5 | PC1-5 (1.0) | - | PC2B-1(2.0) | PC2C-1(2.0) | - |
| Example 6 | PC1-5(0.3) | - | PC2B-1(2.0) | PC2C-1(2.0) | - |
| Comparative Example 2 | - | - | PC2B-1(2.0) | PC2C-1(2.0) | - |

**[Table 3]**

| | First photochromic compound | Second photochromic compound A | Second photochromic compound B | Second photochromic compound C | Other photochromic compounds |
|---|---|---|---|---|---|
| Example 7 | PC1-1(3.0) | PC1-4(1.0) | PC2B-1(0.5) | PC2C-1(0.5) | - |
| Example 8 | PC1-1(3.0) | PC1-4(0.5) | PC2B-1(0.5) | PC2C-1(0.5) | - |
| | | PC1-5(1.0) | | | |
| Example 9 | PC1-5(2.0) | PC1-2 (0.5) | - | PC2C-1(2.0) | - |
| | | PC1-3(0.5) | | | |
| Example 10 | PC1-5(2.0) | PC1-2 (0.5) | PC2B-1(2.0) | - | - |
| | | PC1-3 (0.5) | | | |
| Example 11 | PC1-2(2.5) | PC1-6(1.0) | PC2B-2(0.65) | - | - |
| Example 12 | PC1-8(2.0) | PC1-6 (1.3) | - | PC2C-2 (0.6) | - |
| Example 13 | PC1-9(3.0) | - | - | PC2C-3(1.0) | - |
| | | | | PC2C-4 (0.5) | |
| Comparative Example 3 | - | - | - | PC2C-1(4.0) | - |
| Comparative Example 4 | - | - | PC2B-1(2.0) | - | PC2D-1(2.0) |
| Comparative Example 5 | - | - | - | PC2C-1(2.0) | PC2D-1(2.0) |
| Comparative Example 6 | - | - | PC2B-1(2.5) | - | PC2D-2 (0.55) |
| | | | PC2B-2 (0.65) | | |
| | | | PC2B-3 (0.35) | | |

### <Evaluation test>

### (Solubility evaluation)

For the photochromic polymerizable compositions according to Examples and Comparative Examples, solubility of the photochromic compounds in the polymerizable compounds was visually evaluated.

A degree of dissolution was evaluated according to the following three grades.
1: Complete dissolution, no haze is seen when checked under transmission of light.
2: A thin haze can be seen under transmission of light.
3: A haze can be seen even without transmission of light.

### (1) Initial solubility

This test was performed using "YOKAI-KUN USS-1" manufactured by NIHONSEIKI KAISHA LTD. under the following conditions:
Dissolution temperature: 65 to 70°C
Stirring condition: Stirring dial of 8.
Ultrasonic irradiation condition: Irradiation interval of 3 sec.

Dissolution was checked every 20 minutes up to 1 hour later, a sample was visually checked by transmitting light through the sample, and a degree of dissolution was evaluated. A shorter time taken for the evaluation result to be 1 means higher initial solubility.

### (2) Long-term solubility

After the initial solubility was evaluated, filtration was performed at 25°C under light-shielded conditions using a polyethylene terephthalate (PTFE) filter with a mesh size of 0.5 µm. The resulting photochromic curable composition was stored in a brown bottle container and kept within a thermostatic bath at 40°C. Dissolution (the presence or absence of precipitation) of the photochromic compound was visually evaluated 2 weeks, 1 month, 3 months, 6 months, and 12 months after the start of storage.

The longer a time from the start of storage to precipitation within the thermostatic bath at 40°C, the higher the long-term solubility. The results are shown in Tables 4 to 6.

**[Table 4]**

| | Initial solubility | | | Long-term solubility | | | | |
|---|---|---|---|---|---|---|---|---|
| | 20 min | 40 min | 60 min | 2 weeks | 1 month | 3 months | 6 months | 12 months |
| Example 1 | 1 | 1 | 1 | 1 | 1 | 1 | 1 | 1 |
| Example 2 | 1 | 1 | 1 | 1 | 1 | 1 | 1 | 1 |
| Example 3 | 1 | 1 | 1 | 1 | 1 | 1 | 1 | 1 |
| Example 4 | 2 | 1 | 1 | 1 | 1 | 1 | 1 | 3 |
| Comparative Example 1 | 2 | 2 | 2 | 1 | 1 | 1 | 2 | 3 |

**[Table 5]**

| | Initial solubility | | | Long-term solubility | | | | |
|---|---|---|---|---|---|---|---|---|
| | 20 min | 40 min | 60 min | 2 weeks | 1 month | 3 months | 6 months | 12 months |
| Example 5 | 3 | 2 | 1 | 1 | 1 | 2 | 3 | 3 |
| Example 6 | 3 | 2 | 1 | 1 | 3 | 3 | 3 | 3 |
| Comparative Example 2 | 3 | 2 | 1 | 2 | 3 | 3 | 3 | 3 |

**[Table 6]**

| | Initial solubility | | | Long-term solubility | | | | |
|---|---|---|---|---|---|---|---|---|
| | 20 min | 40 min | 60 min | 2 weeks | 1 month | 3 months | 6 months | 12 months |
| Example 7 | 1 | 1 | 1 | 1 | 1 | 1 | 1 | 2 |
| Example 8 | 1 | 1 | 1 | 1 | 1 | 1 | 1 | 1 |
| Example 9 | 2 | 1 | 1 | 1 | 1 | 1 | 1 | 2 |
| Example 10 | 3 | 1 | 1 | 1 | 1 | 1 | 1 | 2 |
| Example 11 | 1 | 1 | 1 | 1 | 1 | 1 | 1 | 1 |
| Example 12 | 1 | 1 | 1 | 1 | 1 | 1 | 1 | 1 |
| Example 13 | 2 | 1 | 1 | 1 | 1 | 1 | 1 | 1 |
| Comparative Example 3 | 3 | 3 | 2 | 3 | 3 | 3 | 3 | 3 |
| Comparative Example 4 | 3 | 1 | 1 | 1 | 1 | 1 | 2 | 3 |
| Comparative Example 5 | 2 | 1 | 1 | 1 | 1 | 1 | 1 | 2 |
| Comparative Example 6 | 3 | 2 | 1 | 1 | 1 | 1 | 2 | 3 |

### (Evaluation of photochromic performance)

Photochromic laminates were produced using the photochromic curable compositions according to Examples and Comparative Examples, and evaluated for photochromic performance.

First, a thiourethane plastic lens with a center thickness of 2 mm and a refractive index of 1.60 was prepared as an optical substrate. Note that, the thiourethane plastic lens had been alkaline-etched in a 10% sodium hydroxide aqueous solution at 50°C for 5 minutes in advance, and then thoroughly washed with distilled water.

A spin coater (1H-DX2, manufactured by MIKASA CO., LTD) was used to coat a surface of the above-mentioned plastic lens with a moisture-curing primer (product name: TR-SC-P, manufactured by Tokuyama Corporation) at a rotation speed of 70 rpm for 15 seconds, followed by 1000 rpm for 10 seconds. Then, about 2 g of the photochromic curable composition obtained as described above was spin-coated at a rotation speed of 60 rpm for 40 seconds, followed by 600 rpm for 10 to 20 seconds so as to achieve a photochromic coating layer with a film thickness of 40 µm.

The lens of which surface had been coated with the photochromic curable composition (photochromic coating layer) was irradiated with light for 90 seconds in a nitrogen gas atmosphere using a metal halide lamp with an output of 200 mW/cm² to cure the coating. The resultant was then further heated at 110°C for 1 hour to produce a photochromic laminate with a photochromic layer.

### (1) Photochromic property

[1] Maximum absorption wavelength (λmax):
   This is the maximum absorption wavelength after color development determined by a spectrophotometer manufactured by Otsuka Electronics Co., Ltd. (instantaneous multichannel photodetector MCPD3000) and was used as an index of a color tone upon color development.
[2] Color developing density at 23°C (A₂₃):
   This is a difference between an absorbance {ε(300)} after irradiation with light at 23°C for 300 seconds and an absorbance ε(0) when not irradiated with light at the maximum absorption wavelength and was used as an index of a color developing density. The higher this value is, the better a photochromic property is.
[3] Color fading half-life at 23°C [τ1/2 (sec).)]:
   This is a period of time taken by an absorbance at the maximum absorption wavelength of a sample to decrease to 1/2 of {ε(300)-ε(0)} when irradiation is stopped after irradiation with light at 23°C for 300 seconds, and was used as an index of a color fading rate. The shorter this period of time is, the faster the color fading rate is.
[4] Residual ratio (A₁₂₀/A₀ x 100):
   The resulting photochromic plastic lens was subjected to accelerated degradation for 120 hours using a xenon weather meter X25 manufactured by Suga Test Instruments Co., Ltd. Color developing densities were then evaluated as described above before and after a test, and a color developing density before the test (A₀) and a color developing density after the test (A₁₂₀) were measured. A ratio (A₁₂₀/A₀) therebetween was determined as a residual ratio and was used as an index of color development durability. The higher the residual ratio is, the more durable the color development is.

The results are shown in Tables 7 to 9. Note that, for Comparative Examples 1 and 3, only soluble matter was evaluated.

**[Table 7]**

| | Maximum absorption wavelength (nm) | Color developing density at 23°C (-) | Color fading half-life at 23°C (sec) | Residual ratio (%) |
|---|---|---|---|---|
| Example 1 | 464 | 1.58 | 81 | 75% |
| | 569 | 1.10 | 81 | 75% |
| Example 2 | 464 | 1.53 | 84 | 80% |
| | 570 | 1. 05 | 84 | 80% |
| Example 3 | 464 | 1.54 | 85 | 78% |
| | 570 | 1. 05 | 84 | 78% |
| Example 4 | 465 | 1.71 | 95 | 73% |
| | 569 | 1.19 | 94 | 73% |
| Comparative Example 1 | 464 | 1.57 | 89 | 73% |
| | 569 | 1. 09 | 89 | 73% |

**[Table 8]**

| | Maximum absorption wavelength (nm) | Color developing density at 23°C (-) | Color fading half-life at 23°C (sec) | Residual ratio (%) |
|---|---|---|---|---|
| Example 5 | 464 | 1.55 | 87 | 78% |
| | 568 | 1.14 | 87 | 82% |
| Example 6 | 464 | 1.40 | 88 | 73% |
| | 568 | 1. 04 | 88 | 76% |
| Comparative Example 2 | 464 | 1.40 | 88 | 72% |
| | 568 | 1. 04 | 88 | 77% |

**[Table 9]**

| | Maximum absorption wavelength (nm) | Color developing density at 23°C (-) | Color fading half-life at 23°C (sec) | Residual ratio (%) |
|---|---|---|---|---|
| Example 7 | 464 | 1.62 | 77 | 82% |
| | 569 | 1.14 | 77 | 83% |
| Example 8 | 464 | 1.70 | 81 | 81% |
| | 569 | 1.19 | 81 | 82% |
| Example 9 | 463 | 1.48 | 78 | 80% |
| | 568 | 1. 08 | 75 | 83% |
| Example 10 | 465 | 1.66 | 94 | 76% |
| | 569 | 1.15 | 94 | 76% |
| Example 11 | 462 | 1.21 | 100 | 75% |
| | 583 | 1.27 | 102 | 69% |
| Example 12 | 458 | 0.98 | 80 | 80% |
| | 613 | 1. 09 | 82 | 82% |
| Example 13 | 483 | 0.97 | 40 | 76% |
| | 610 | 0.97 | 40 | 73% |
| Comparative Example 3 | 461 | 1. 07 | 73 | 89% |
| | 566 | 0.86 | 72 | 89% |
| Comparative Example 4 | 465 | 1. 03 | 66 | 50% |
| | 570 | 0.76 | 66 | 51% |
| Comparative Example 5 | 463 | 0.88 | 56 | 56% |
| | 569 | 0.70 | 56 | 59% |
| Comparative Example 6 | 461 | 1.26 | 96 | 70% |
| | 582 | 1.24 | 99 | 57% |

Preferred embodiments of the present disclosure will be described in addition.
[1] A photochromic composition including:
   a first photochromic compound represented by Formula (1) below; and
   at least one second photochromic compound selected from the group consisting of a second photochromic compound A represented by Formula (1) and having a different structure from the first photochromic compound, a second photochromic compound B represented by Formula (2) below, and a second photochromic compound C represented by Formula (3) below: 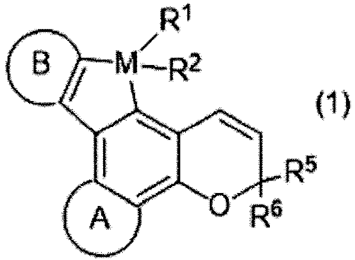 in Formula (1),
   M is C, Si, or Ge;
   Ring A is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
   Ring B is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings; and
   R¹ and R² are each independently a substituted or unsubstituted alkyl group or a group represented by Formula (2a) below and have different structures from each other:

      -Q¹-(X¹Q²)a-X²Q³ (2a)
   in Formula (2a),
   Q¹ is an alkylene group or a haloalkylene group;
   Q² is an alkylene group or a haloalkylene group;
   Q³ is an alkyl group or a haloalkyl group;
   X¹ and X² are each independently O, S, NR⁷⁰⁰, PR⁷⁰¹, or P(=O); R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group;
   a is an integer of 0 or more and 10 or less; and
   R⁵ and R⁶ are each independently a hydrogen atom or a substituent: 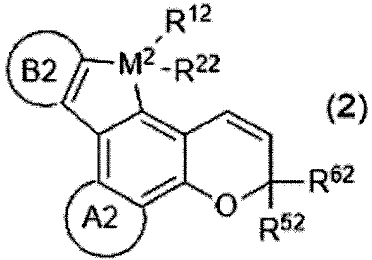
   in Formula (2),
   M² is C, Si, or Ge;
   Ring A2 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
   Ring B2 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
   R¹² and R²² are each a substituted or unsubstituted alkyl group or a group represented by Formula (2a) above and have the same structure as each other; and
   R⁵² and R⁶² are each independently a hydrogen atom or a substituent: 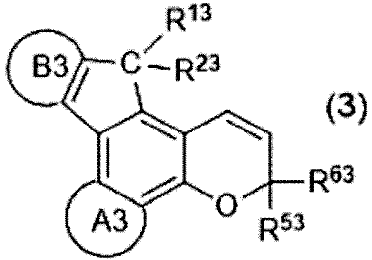
   in Formula (3),
   Ring A3 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
   Ring B3 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
   R¹³ and R²³ are taken together with a carbon atom to which they are attached to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the above-mentioned heterocyclic ring; and
   R⁵³ and R⁶³ are each independently a hydrogen atom or a substituent.
[2] The photochromic composition according to [1], in which, in Formula (1), R¹ and R² are each independently a linear or branched alkyl group having 1 to 20 carbon atoms, an alkyl group having 1 to 20 carbon atoms and substituted with a halogen atom, or a group represented by Formula (2b) below:

   -Q¹-(OQ²)a-OQ³ (2b)

   in Formula (2b), Q¹, Q², and Q³ are the same as for Formula (2a).
[3] The photochromic composition according to [1] or [2], in which the first photochromic compound includes a compound represented by Formula (5) below: 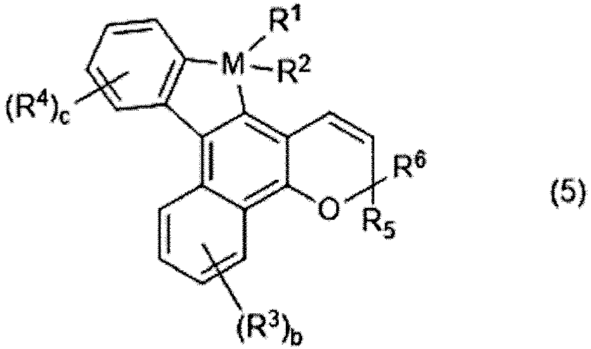 in Formula (5),
   R¹, R², and M are each the same as for Formula (1);
   R³ and R⁴ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) below, or a group represented by Formula (X3) below;
   b is an integer of 0 or more and 4 or less; c is an integer of 0 or more and 4 or less;
   when b is an integer of 2 or more and 4 or less, a plurality of R³s may be the same as or different from each other;
   when c is an integer of 2 or more and 4 or less, a plurality of R⁴s may be the same as or different from each other;
   when b is an integer of 2 or more and 4 or less and adjacent R³s are present, two adjacent R³s may be taken together with a carbon atom to which R³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings;
   when c is an integer of 2 or more and 4 or less and adjacent R⁴s are present, two adjacent R⁴s may be taken together with a carbon atom to which R⁴s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
   R⁵ and R⁶ are each independently a substituted or unsubstituted aryl group or a substituted or unsubstituted heteroaryl group; 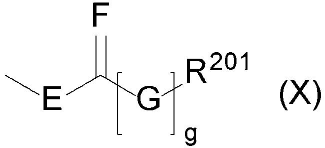
   in Formula (X),
   E is an oxygen atom or NR¹⁰¹ in which R¹⁰¹ is a hydrogen atom or an alkyl group;
   F is an oxygen atom or a sulfur atom;
   G is an oxygen atom, a sulfur atom, or NR²⁰² wherein R²⁰² is a hydrogen atom, an alkyl group, a cycloalkyl group, an aryl group, or a heteroaryl group;
   g is 0 or 1;
   R²⁰¹ is a hydrogen atom, an alkyl group, a cycloalkyl group, an aryl group, or a heteroaryl group; and
   when G is an oxygen atom or a sulfur atom, R²⁰¹ is a group other than a hydrogen atom;

   L¹-R⁴⁰⁰ (X3)

   in Formula (X3),
   R⁴⁰⁰ is a hydrogen atom, an alkyl group, an aryl group, a polymerizable group, a photochromic group, or a silyl group having an alkyl group, an alkoxy group, or an aryl group as a substituent; and
   L¹ is a group represented by Formula (X2) below: 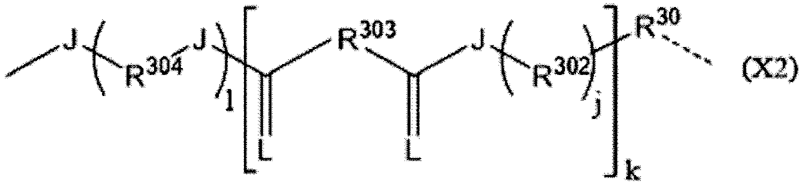
   in Formula (X2), R³⁰ is a group represented by Formula (X2a) below; 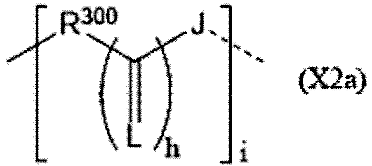
   in Formulae (X2) and (X2a),
   J is a divalent group, each independently being directly attached, a substituted or unsubstituted methylene group, an oxygen atom, a sulfur atom, or NR³⁰¹ in which R³⁰¹ is a hydrogen atom or an alkyl group;
   L is an oxygen atom or a sulfur atom;
   R³⁰⁰ is an alkylene group, or a silylene group having an alkyl group or an aryl group as a substituent;
   R³⁰², R³⁰³, and R³⁰⁴ are each independently an alkylene group;
   h, j, k, and l are each independently 0 or 1;
   i is an integer from 0 or more and 200 or less; when i is 2 or more, a plurality of R³⁰s may be the same as or different from each other; and a dashed line represents an attachment to R⁴⁰⁰.
[4] The photochromic composition according to [3], in which the first photochromic compound includes a compound represented by Formula (6) below: 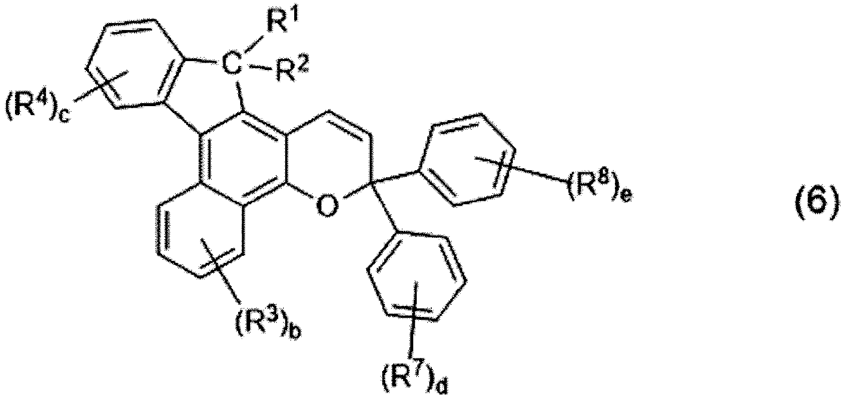 in Formula (6),
   R¹, R², R³, R⁴, b, and c are each the same as for Formula (5) above;
   R⁷ and R⁸ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above;
   d is an integer of 0 or more and 5 or less; e is an integer of 0 or more and 5 or less;
   when d is an integer of 2 or more and 5 or less, a plurality of R⁷s may be the same as or different from each other;
   when e is an integer of 2 or more and 5 or less, a plurality of R⁸s may be the same as or different from each other;
   when d is an integer of 2 or more and 5 or less and adjacent R⁷s are present, two adjacent R⁷s may be taken together with a carbon atom to which R⁷s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
   when e is an integer of 2 or more and 5 or less and adjacent R⁸s are present, two adjacent R⁸s may be taken together with a carbon atom to which R⁸s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings.
[5] The photochromic composition according to any one of [1] to [4], in which the second photochromic compound includes a second photochromic compound B represented by Formula (7) below: 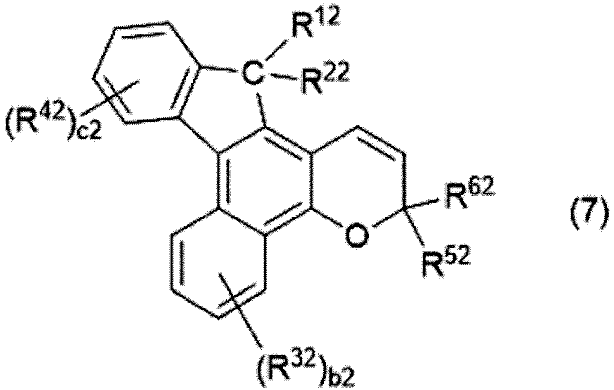 in Formula (7),
   R¹² and R²² are each the same as for Formula (2);
   R³² and R⁴² are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) below, or a group represented by Formula (X3) below;
   b2 is an integer of 0 or more and 4 or less; c2 is an integer of 0 or more and 4 or less;
   when b2 is an integer of 2 or more and 4 or less, a plurality of R³²s may be the same as or different from each other;
   when c2 is an integer of 2 or more and 4 or less, a plurality of R⁴²s may be the same as or different from each other;
   when b2 is an integer of 2 or more and 4 or less and adjacent R³²s are present, two adjacent R³²s may be taken together with a carbon atom to which R³²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings;
   when c2 is an integer of 2 or more and 4 or less and adjacent R⁴²s are present, two adjacent R⁴²s may be taken together with a carbon atom to which R⁴²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
   R⁵² and R⁶² are each independently a substituted or unsubstituted aryl group or a substituted or unsubstituted heteroaryl group; 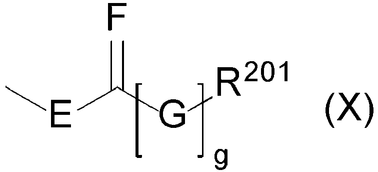
   in Formula (X),
   E is an oxygen atom or NR¹⁰¹ in which R¹⁰¹ is a hydrogen atom or an alkyl group;
   F is an oxygen atom or a sulfur atom;
   G is an oxygen atom, a sulfur atom, or NR²⁰² in which R²⁰² is a hydrogen atom, an alkyl group, a cycloalkyl group, an aryl group, or a heteroaryl group;
   g is 0 or 1;
   R²⁰¹ is a hydrogen atom, an alkyl group, a cycloalkyl group, an aryl group, or a heteroaryl group; and
   when G is an oxygen atom or a sulfur atom, R²⁰¹ is a group other than a hydrogen atom;

   L¹-R⁴⁰⁰ (X3)

   in Formula (X3),
   R⁴⁰⁰ is a hydrogen atom, an alkyl group, an aryl group, a polymerizable group, a photochromic group, or a silyl group having an alkyl group, an alkoxy group, or an aryl group as a substituent; and
   L¹ is a group represented by Formula (X2) below: 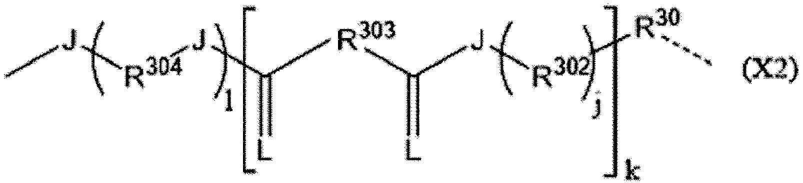
   in Formula (X2), R³⁰ is a group represented by Formula (X2a) below; 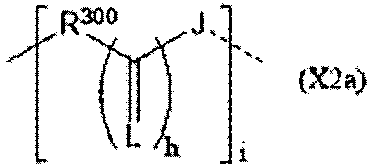
   in Formulae (X2) and (X2a),
   J is a divalent group, each independently being directly attached, a substituted or unsubstituted methylene group, an oxygen atom, a sulfur atom, or NR³⁰¹ in which R³⁰¹ is a hydrogen atom or an alkyl group;
   L is an oxygen atom or a sulfur atom;
   R³⁰⁰ is an alkylene group, or a silylene group having an alkyl group or an aryl group as a substituent;
   R³⁰², R³⁰³, and R³⁰⁴ are each independently an alkylene group;
   h, j, k, and l are each independently 0 or 1;
   i is an integer from 0 or more and 200 or less; when i is 2 or more, a plurality of R³⁰s may be the same as or different from each other; and a dashed line represents an attachment to R⁴⁰⁰.
[6] The photochromic composition according to [5], in which the second photochromic compound B is represented by Formula (8) below: 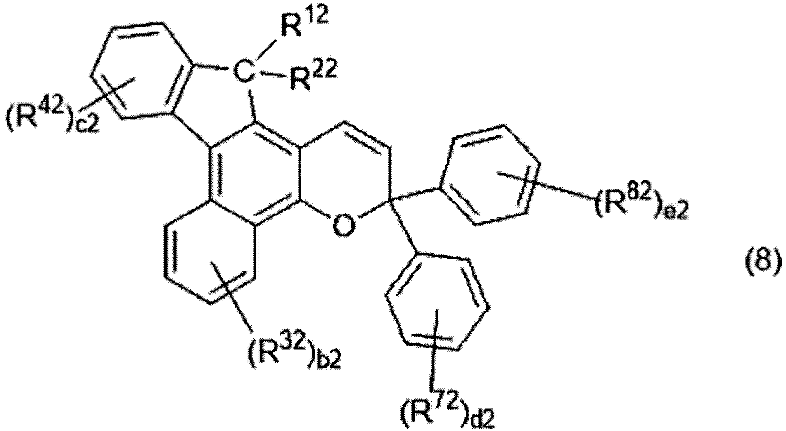 in Formula (8),
   R¹², R²², R³², R⁴², b2, and c2 are each the same as for Formula (7) above;
   R⁷² and R⁸² are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above;
   d2 is an integer of 0 or more and 5 or less; e2 is an integer of 0 or more and 5 or less;
   when d2 is an integer of 2 or more and 5 or less, a plurality of R⁷²s may be the same as or different from each other;
   when e2 is an integer of 2 or more and 5 or less, a plurality of R⁸²s may be the same as or different from each other;
   when d2 is an integer of 2 or more and 5 or less and adjacent R⁷²s are present, two adjacent R⁷²s may be taken together with a carbon atom to which R⁷²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
   when e2 is an integer of 2 or more and 5 or less and adjacent R⁸²s are present, two adjacent R⁸²s may be taken together with a carbon atom to which R⁸²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings.
[7] The photochromic composition according to any one of [1] to [6], in which the second photochromic compound includes a second photochromic compound C represented by Formula (9) below: 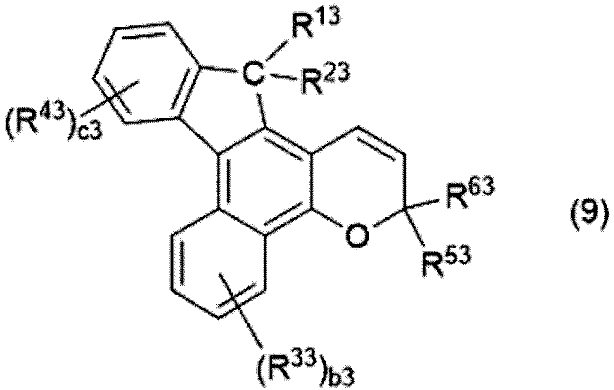 in Formula (9),
   R¹³ and R²³ are each the same as for Formula (3);
   R³³ and R⁴³ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) below, or a group represented by Formula (X3) below;
   b3 is an integer of 0 or more and 4 or less; c3 is an integer of 0 or more and 4 or less;
   when b3 is an integer of 2 or more and 4 or less, a plurality of R³³s may be the same as or different from each other;
   when c3 is an integer of 2 or more and 4 or less, a plurality of R⁴³s may be the same as or different from each other;
   when b3 is an integer of 2 or more and 4 or less and adjacent R³³s are present, two adjacent R³³s may be taken together with a carbon atom to which R³³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings;
   when c3 is an integer of 2 or more and 4 or less and adjacent R⁴³s are present, two adjacent R⁴³s may be taken together with a carbon atom to which R⁴³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
   R⁵³ and R⁶³ are each independently a substituted or unsubstituted aryl group or a substituted or unsubstituted heteroaryl group; 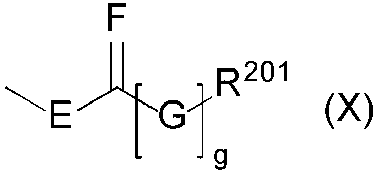
   in Formula (X),
   E is an oxygen atom or NR¹⁰¹ in which R¹⁰¹ is a hydrogen atom or an alkyl group;
   F is an oxygen atom or a sulfur atom;
   G is an oxygen atom, a sulfur atom, or NR²⁰² in which R²⁰² is a hydrogen atom, an alkyl group, a cycloalkyl group, an aryl group, or a heteroaryl group;
   g is 0 or 1;
   R²⁰¹ is a hydrogen atom, an alkyl group, a cycloalkyl group, an aryl group, or a heteroaryl group; and
   when G is an oxygen atom or a sulfur atom, R²⁰¹ is a group other than a hydrogen atom;

   L¹-R⁴⁰⁰ (X3)

   in Formula (X3),
   R⁴⁰⁰ is a hydrogen atom, an alkyl group, an aryl group, a polymerizable group, a photochromic group, or a silyl group having an alkyl group, an alkoxy group, or an aryl group as a substituent; and
   L¹ is a group represented by Formula (X2) below: 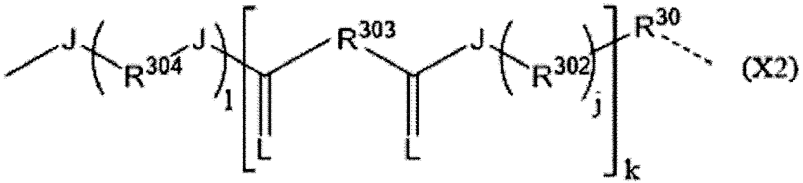
   in Formula (X2), R³⁰ is a group represented by Formula (X2a) below; 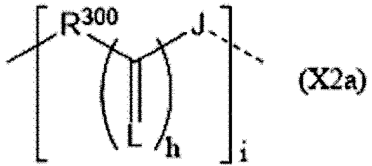
   in Formulae (X2) and (X2a),
   J is a divalent group, each independently being directly attached, a substituted or unsubstituted methylene group, an oxygen atom, a sulfur atom, or NR³⁰¹ in which R³⁰¹ is a hydrogen atom or an alkyl group;
   L is an oxygen atom or a sulfur atom;
   R³⁰⁰ is an alkylene group, or a silylene group having an alkyl group or an aryl group as a substituent;
   R³⁰², R³⁰³, and R³⁰⁴ are each independently an alkylene group;
   h, j, k, and l are each independently 0 or 1;
   i is an integer from 0 or more and 200 or less; when i is 2 or more, a plurality of R³⁰s may be the same as or different from each other; and a dashed line represents an attachment to R⁴⁰⁰.
[8] The photochromic composition according to [7], in which the second photochromic compound C is represented by Formula (10) below: 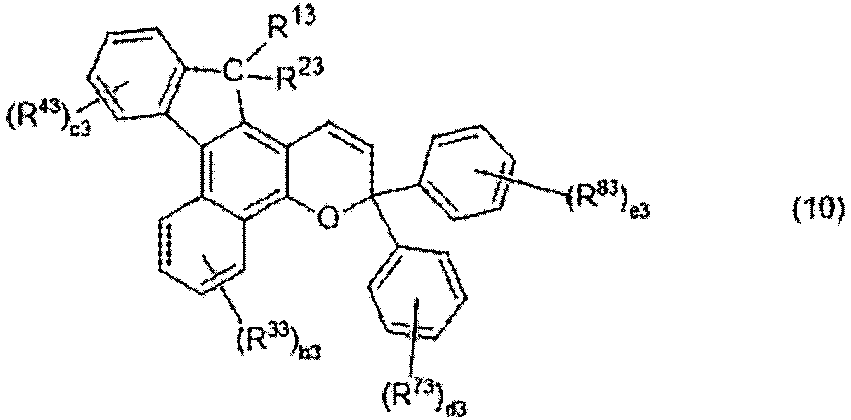 in Formula (10),
   R¹³, R²³, R³³, R⁴³, b3, and c3 are each the same as for Formula (9) above;
   R⁷³ and R⁸³ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above;
   d3 is an integer of 0 or more and 5 or less; e3 is an integer of 0 or more and 5 or less;
   when d3 is an integer of 2 or more and 5 or less, a plurality of R⁷³s may be the same as or different from each other;
   when e3 is an integer of 2 or more and 5 or less, a plurality of R⁸³s may be the same as or different from each other;
   when d3 is an integer of 2 or more and 5 or less and adjacent R⁷³s are present, two adjacent R⁷³s may be taken together with a carbon atom to which R⁷³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
   when e3 is an integer of 2 or more and 5 or less and adjacent R⁸³s are present, two adjacent R⁸³s may be taken together with a carbon atom to which R⁸³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings.
[9] The photochromic composition according to any one of [1] to [8], in which a content of the first photochromic compound is 1% by mass or more and 99% by mass or less.
[10] The photochromic composition according to any one of [1] to [9], in which a content of the first photochromic compound is 1% by mass or more and 99% by mass or less and a content of the second photochromic compound B is 1% by mass or more and 50% by mass or less.
[11] The photochromic composition according to any one of [1] to [10], in which a content of the first photochromic compound is 1% by mass or more and 99% by mass or less and a content of the second photochromic compound C is 1% by mass or more and 50% by mass or less.
[12] The photochromic composition according to any one of [1] to [11], in which a total content of the first photochromic compound, the second photochromic compound A, the second photochromic compound B, and the second photochromic compound C is 80% by mass or more and 100% by mass or less.
[13] The photochromic composition according to any one of [1] to [12], in which the photochromic composition consists of the first photochromic compound, the second photochromic compound A, the second photochromic compound B, and the second photochromic compound C, a content of the first photochromic compound is 1% by mass or more and 99% by mass or less, a content of the second photochromic compound A is 1% by mass or more and 99% by mass or less, a content of the second photochromic compound B is 1% by mass or more and 99% by mass or less, and a content of the second photochromic compound C is 1% by mass or more and 99% by mass or less.
[14] The photochromic composition according to any one of [1] to [13], in which the photochromic composition includes the second photochromic compound A.
[15] A curable composition including:
   the photochromic composition according to any one of [1] to [14]; and
   at least one selected from the group consisting of a radical polymerizable monomer, a cationic polymerizable monomer, a compound having a polymerization reactive group, and a (thio)urethane(urea) polymer.
[16] A cured product of the curable composition according to [15].
[17] An optical article including the cured product according to [16].
[18] A lens including the photochromic composition according to any one of [1] to [14].
[19] Eyeglasses including the lens according to [18].

## Claims

1. A photochromic composition comprising:
a first photochromic compound represented by Formula (1) below; and
at least one second photochromic compound selected from the group consisting of a second photochromic compound A represented by Formula (1) and having a different structure from the first photochromic compound, a second photochromic compound B represented by Formula (2) below, and a second photochromic compound C represented by Formula (3) below: 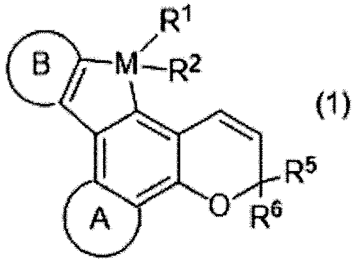
in Formula (1),
M is C, Si, or Ge;
Ring A is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
Ring B is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings; and
R¹ and R² are each independently a substituted or unsubstituted alkyl group or a group represented by Formula (2a) below and have different structures from each other:
-Q¹-(X¹Q²)a-X²Q³ (2a)
in Formula (2a),
Q¹ is an alkylene group or a haloalkylene group;
Q² is an alkylene group or a haloalkylene group;
Q³ is an alkyl group or a haloalkyl group;
X¹ and X² are each independently O, S, NR⁷⁰⁰, PR⁷⁰¹, or P (=O) ;
R⁷⁰⁰ and R⁷⁰¹ are each independently a hydrogen atom, a substituted or unsubstituted alkyl group, a substituted or unsubstituted cycloalkyl group, a substituted or unsubstituted aryl group, or a substituted or unsubstituted heteroaryl group;
a is an integer of 0 or more and 10 or less; and
R⁵ and R⁶ are each independently a hydrogen atom or a substituent: 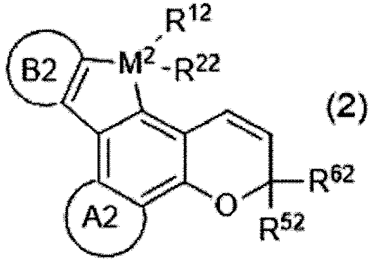
in Formula (2),
M² is C, Si, or Ge;
Ring A2 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
Ring B2 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
R¹² and R²² are each a substituted or unsubstituted alkyl group or a group represented by Formula (2a) above and have the same structure as each other; and
R⁵² and R⁶² are each independently a hydrogen atom or a substituent: 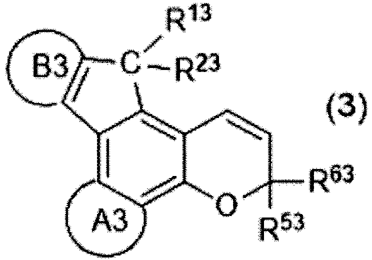
in Formula (3),
Ring A3 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
Ring B3 is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
R¹³ and R²³ are taken together with a carbon atom to which they are attached to form a substituted or unsubstituted aliphatic ring having 3 to 20 ring member carbon atoms, a substituted or unsubstituted fused polycyclic ring in which an aromatic hydrocarbon ring or an aromatic heterocyclic ring is fused to the aliphatic ring, a substituted or unsubstituted heterocyclic ring having 3 to 20 ring member atoms, or a substituted or unsubstituted fused polycyclic ring in which an aromatic ring or an aromatic heterocyclic ring is fused to the heterocyclic ring; and
R⁵³ and R⁶³ are each independently a hydrogen atom or a substituent.

2. The photochromic composition according to claim 1, wherein, in Formula (1), R¹ and R² are each independently a linear or branched alkyl group having 1 to 20 carbon atoms, an alkyl group having 1 to 20 carbon atoms and substituted with a halogen atom, or a group represented by Formula (2b) below:
-Q1-(OQ²)ₐ-OQ³ (2b)
in Formula (2b), Q¹, Q², and Q³ are the same as for Formula (2a).

3. The photochromic composition according to claim 1, wherein the first photochromic compound includes a compound represented by Formula (5) below: 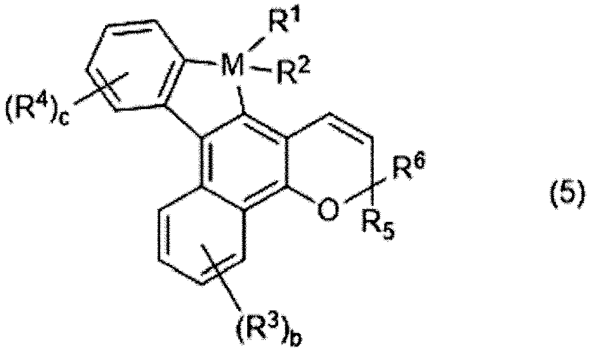
in Formula (5),
R¹, R², and M are each the same as for Formula (1);
R³ and R⁴ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) below, or a group represented by Formula (X3) below;
b is an integer of 0 or more and 4 or less; c is an integer of 0 or more and 4 or less;
when b is an integer of 2 or more and 4 or less, a plurality of R³s may be the same as or different from each other;
when c is an integer of 2 or more and 4 or less, a plurality of R⁴s may be the same as or different from each other;
when b is an integer of 2 or more and 4 or less and adjacent R³s are present, two adjacent R³s may be taken together with a carbon atom to which R³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings;
when c is an integer of 2 or more and 4 or less and adjacent R⁴s are present, two adjacent R⁴s may be taken together with a carbon atom to which R⁴s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
R⁵ and R⁶ are each independently a substituted or unsubstituted aryl group or a substituted or unsubstituted heteroaryl group; 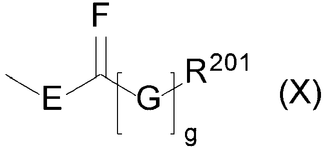
in Formula (X),
E is an oxygen atom or NR¹⁰¹ wherein R¹⁰¹ is a hydrogen atom or an alkyl group;
F is an oxygen atom or a sulfur atom;
G is an oxygen atom, a sulfur atom, or NR²⁰² wherein R²⁰² is a hydrogen atom, an alkyl group, a cycloalkyl group, an aryl group, or a heteroaryl group;
g is 0 or 1;
R²⁰¹ is a hydrogen atom, an alkyl group, a cycloalkyl group, an aryl group, or a heteroaryl group; and
when G is an oxygen atom or a sulfur atom, R²⁰¹ is a group other than a hydrogen atom;
L¹-R⁴⁰⁰ (X3)
in Formula (X3),
R⁴⁰⁰ is a hydrogen atom, an alkyl group, an aryl group, a polymerizable group, a photochromic group, or a silyl group having an alkyl group, an alkoxy group, or an aryl group as a substituent; and
L¹ is a group represented by Formula (X2) below: 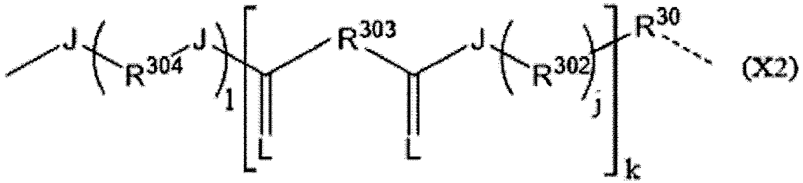
in Formula (X2), R³⁰ is a group represented by Formula (X2a) below; 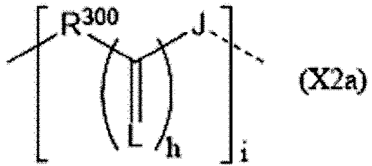
in Formulae (X2) and (X2a),
J is a divalent group, each independently being directly attached, a substituted or unsubstituted methylene group, an oxygen atom, a sulfur atom, or NR³⁰¹ wherein R³⁰¹ is a hydrogen atom or an alkyl group;
L is an oxygen atom or a sulfur atom;
R³⁰⁰ is an alkylene group, or a silylene group having an alkyl group or an aryl group as a substituent;
R³⁰², R³⁰³, and R³⁰⁴ are each independently an alkylene group;
h, j, k, and l are each independently 0 or 1;
i is an integer from 0 or more and 200 or less; when i is 2 or more, a plurality of R³⁰s may be the same as or different from each other; and a dashed line represents an attachment to R⁴⁰⁰.

4. The photochromic composition according to claim 3, wherein the first photochromic compound comprises a compound represented by Formula (6) below: 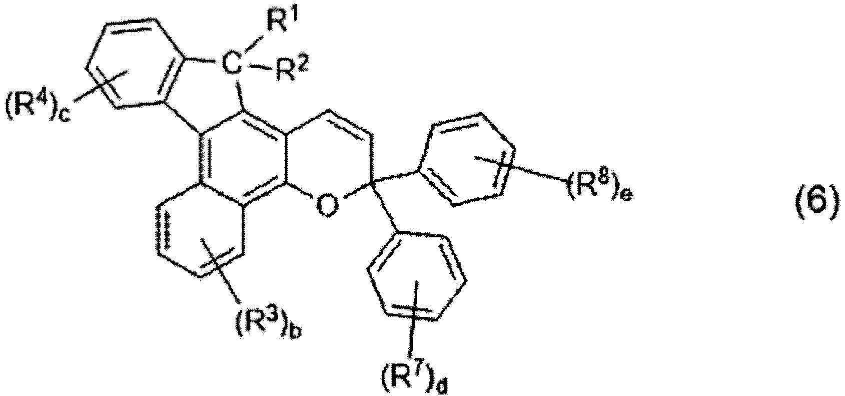
in Formula (6),
R¹, R², R³, R⁴, b, and c are each the same as for Formula (5) above;
R⁷ and R⁸ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above;
d is an integer of 0 or more and 5 or less; e is an integer of 0 or more and 5 or less;
when d is an integer of 2 or more and 5 or less, a plurality of R⁷s may be the same as or different from each other;
when e is an integer of 2 or more and 5 or less, a plurality of R⁸s may be the same as or different from each other;
when d is an integer of 2 or more and 5 or less and adjacent R⁷s are present, two adjacent R⁷s may be taken together with a carbon atom to which R⁷s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
when e is an integer of 2 or more and 5 or less and adjacent R⁸s are present, two adjacent R⁸s may be taken together with a carbon atom to which R⁸s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings.

5. The photochromic composition according to claim 1, wherein the second photochromic compound comprises a second photochromic compound B represented by Formula (7) below: 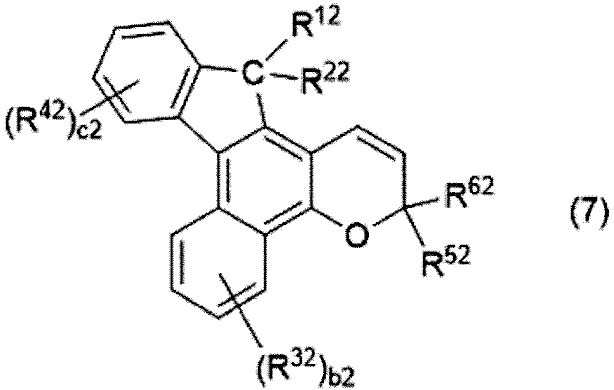
in Formula (7),
R¹² and R²² are each the same as for Formula (2);
R³² and R⁴² are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) below, or a group represented by Formula (X3) below;
b2 is an integer of 0 or more and 4 or less; c2 is an integer of 0 or more and 4 or less;
when b2 is an integer of 2 or more and 4 or less, a plurality of R³²s may be the same as or different from each other;
when c2 is an integer of 2 or more and 4 or less, a plurality of R⁴²s may be the same as or different from each other;
when b2 is an integer of 2 or more and 4 or less and adjacent R³² are present, two adjacent R³²s may be taken together with a carbon atom to which R³²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings;
when c2 is an integer of 2 or more and 4 or less and adjacent R⁴²s are present, two adjacent R⁴²s may be taken together with a carbon atom to which R⁴²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
R⁵² and R⁶² are each independently a substituted or unsubstituted aryl group or a substituted or unsubstituted heteroaryl group; 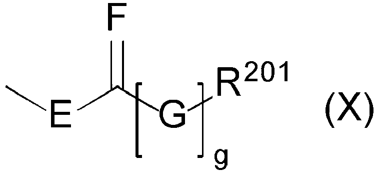
in Formula (X),
E is an oxygen atom or NR¹⁰¹ wherein R¹⁰¹ is a hydrogen atom or an alkyl group;
F is an oxygen atom or a sulfur atom;
G is an oxygen atom, a sulfur atom, or NR²⁰² wherein R²⁰² is a hydrogen atom, an alkyl group, a cycloalkyl group, an aryl group, or a heteroaryl group;
g is 0 or 1;
R²⁰¹ is a hydrogen atom, an alkyl group, a cycloalkyl group, an aryl group, or a heteroaryl group; and
when G is an oxygen atom or a sulfur atom, R²⁰¹ is a group other than a hydrogen atom;
L¹-R⁴⁰⁰ (X3)
in Formula (X3),
R⁴⁰⁰ is a hydrogen atom, an alkyl group, an aryl group, a polymerizable group, a photochromic group, or a silyl group having an alkyl group, an alkoxy group, or an aryl group as a substituent; and
L¹ is a group represented by Formula (X2) below: 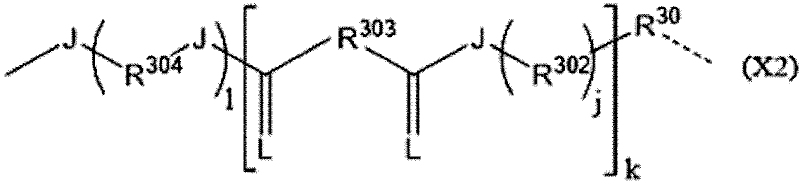
in Formula (X2), R³⁰ is a group represented by Formula (X2a) below; 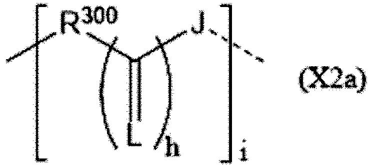
in Formulae (X2) and (X2a),
J is a divalent group, each independently being directly attached, a substituted or unsubstituted methylene group, an oxygen atom, a sulfur atom, or NR³⁰¹ wherein R³⁰¹ is a hydrogen atom or an alkyl group;
L is an oxygen atom or a sulfur atom;
R³⁰⁰ is an alkylene group, or a silylene group having an alkyl group or an aryl group as a substituent;
R³⁰², R³⁰³, and R³⁰⁴ are each independently an alkylene group;
h, j, k, and l are each independently 0 or 1;
i is an integer from 0 or more and 200 or less; when i is 2 or more, a plurality of R³⁰s may be the same as or different from each other; and a dashed line represents an attachment to R⁴⁰⁰.

6. The photochromic composition according to claim 5, wherein the second photochromic compound B is represented by Formula (8) below: 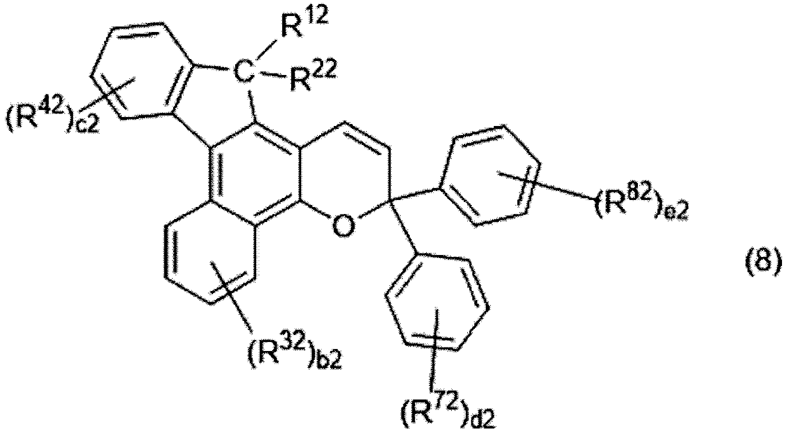
in Formula (8),
R¹², R²², R³², R⁴², b2, and c2 are each the same as for Formula (7) above;
R⁷² and R⁸² are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above;
d2 is an integer of 0 or more and 5 or less; e2 is an integer of 0 or more and 5 or less;
when d2 is an integer of 2 or more and 5 or less, a plurality of R⁷²s may be the same as or different from each other;
when e2 is an integer of 2 or more and 5 or less, a plurality of R⁸²s may be the same as or different from each other;
when d2 is an integer of 2 or more and 5 or less and adjacent R⁷²s are present, two adjacent R⁷²s may be taken together with a carbon atom to which R⁷²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
when e2 is an integer of 2 or more and 5 or less and adjacent R⁸²s are present, two adjacent R⁸²s may be taken together with a carbon atom to which R⁸²s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings.

7. The photochromic composition according to claim 1, wherein the second photochromic compound includes a second photochromic compound C represented by Formula (9) below: 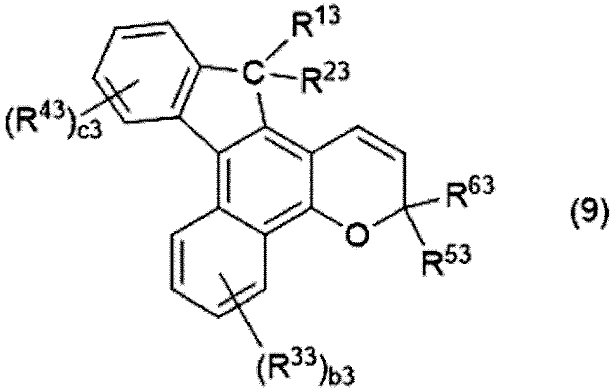
in Formula (9),
R¹³ and R²³ are each the same as for Formula (3);
R³³ and R⁴³ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) below, or a group represented by Formula (X3) below;
b3 is an integer of 0 or more and 4 or less; c3 is an integer of 0 or more and 4 or less;
when b3 is an integer of 2 or more and 4 or less, a plurality of R³³s may be the same as or different from each other;
when c3 is an integer of 2 or more and 4 or less, a plurality of R⁴³s may be the same as or different from each other;
when b3 is an integer of 2 or more and 4 or less and adjacent R³³s are present, two adjacent R³³s may be taken together with a carbon atom to which R³³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings;
when c3 is an integer of 2 or more and 4 or less and adjacent R⁴³s are present, two adjacent R⁴³s may be taken together with a carbon atom to which R⁴³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
R⁵³ and R⁶³ are each independently a substituted or unsubstituted aryl group or a substituted or unsubstituted heteroaryl group; 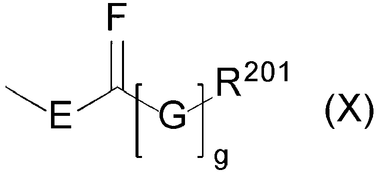
in Formula (X),
E is an oxygen atom or NR¹⁰¹ wherein R¹⁰¹ is a hydrogen atom or an alkyl group;
F is an oxygen atom or a sulfur atom;
G is an oxygen atom, a sulfur atom, or NR²⁰² wherein R²⁰² is a hydrogen atom, an alkyl group, a cycloalkyl group, an aryl group, or a heteroaryl group;
g is 0 or 1;
R²⁰¹ is a hydrogen atom, an alkyl group, a cycloalkyl group, an aryl group, or a heteroaryl group; and
when G is an oxygen atom or a sulfur atom, R²⁰¹ is a group other than a hydrogen atom;
L¹-R⁴⁰⁰ (X3)
in Formula (X3),
R⁴⁰⁰ is a hydrogen atom, an alkyl group, an aryl group, a polymerizable group, a photochromic group, or a silyl group having an alkyl group, an alkoxy group, or an aryl group as a substituent; and
L¹ is a group represented by Formula (X2) below: 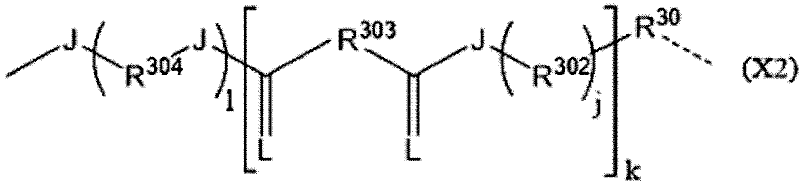
in Formula (X2), R³⁰ is a group represented by Formula (X2a) below; 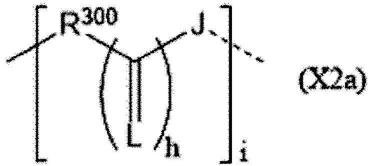
in Formulae (X2) and (X2a),
J is a divalent group, each independently being directly attached, a substituted or unsubstituted methylene group, an oxygen atom, a sulfur atom, or NR³⁰¹ wherein R³⁰¹ is a hydrogen atom or an alkyl group;
L is an oxygen atom or a sulfur atom;
R³⁰⁰ is an alkylene group, or a silylene group having an alkyl group or an aryl group as a substituent;
R³⁰², R³⁰³, and R³⁰⁴ are each independently an alkylene group;
h, j, k, and l are each independently 0 or 1;
i is an integer from 0 or more and 200 or less; when i is 2 or more, a plurality of R³⁰s may be the same as or different from each other; and a dashed line represents an attachment to R⁴⁰⁰.

8. The photochromic composition according to claim 7, wherein the second photochromic compound C is represented by Formula (10) below: 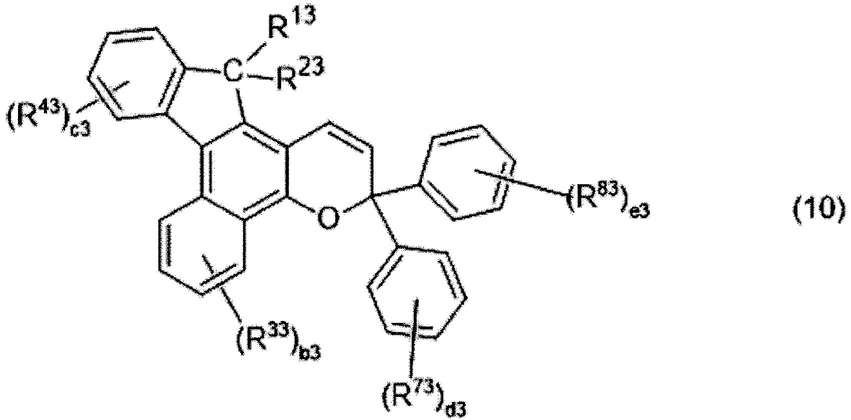
in Formula (10),
R¹³, R²³, R³³, R⁴³, b3, and c3 are each the same as for Formula (9) above;
R⁷³ and R⁸³ are each independently a hydroxy group, a substituted or unsubstituted alkyl group, a haloalkyl group, a substituted or unsubstituted cycloalkyl group, a substituted or unsubstituted alkoxy group, a substituted or unsubstituted amino group, a substituted or unsubstituted heterocyclic group, a cyano group, a halogen atom, an alkylthio group, a substituted or unsubstituted arylthio group, a nitro group, a formyl group, a hydroxycarbonyl group, an alkylcarbonyl group, an alkoxycarbonyl group, a substituted or unsubstituted aralkyl group, a substituted or unsubstituted aralkoxy group, a substituted or unsubstituted aryloxy group, a substituted or unsubstituted aryl group, a substituted or unsubstituted heteroaryl group, a thiol group, an alkoxyalkylthio group, a haloalkylthio group, a substituted or unsubstituted cycloalkylthio group, a substituted or unsubstituted silyl group, a substituted or unsubstituted oxysilyl group, a group represented by Formula (2a) above, a group represented by Formula (X) above, or a group represented by Formula (X3) above;
d3 is an integer of 0 or more and 5 or less; e3 is an integer of 0 or more and 5 or less;
when d3 is an integer of 2 or more and 5 or less, a plurality of R⁷³s may be the same as or different from each other;
when e3 is an integer of 2 or more and 5 or less, a plurality of R⁸³s may be the same as or different from each other;
when d3 is an integer of 2 or more and 5 or less and adjacent R⁷³s are present, two adjacent R⁷³s may be taken together with a carbon atom to which R⁷³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings; and
when e3 is an integer of 2 or more and 5 or less and adjacent R⁸³s are present, two adjacent R⁸³s may be taken together with a carbon atom to which R⁸³s are attached to form a substituted or unsubstituted aliphatic ring, a substituted or unsubstituted aliphatic heterocyclic ring, a substituted or unsubstituted aromatic ring, a substituted or unsubstituted aromatic heterocyclic ring, or a substituted or unsubstituted fused polycyclic ring in which an aromatic ring or an aromatic heterocyclic ring is fused to any of the above-mentioned rings.

9. The photochromic composition according to claim 1, wherein a content of the first photochromic compound is 1% by mass or more and 99% by mass or less.

10. The photochromic composition according to claim 1, wherein a content of the first photochromic compound is 1% by mass or more and 99% by mass or less and a content of the second photochromic compound B is 1% by mass or more and 50% by mass or less.

11. The photochromic composition according to claim 1, wherein a content of the first photochromic compound is 1% by mass or more and 99% by mass or less and a content of the second photochromic compound C is 1% by mass or more and 50% by mass or less.

12. The photochromic composition according to claim 1, wherein a total content of the first photochromic compound, the second photochromic compound A, the second photochromic compound B, and the second photochromic compound C is 80% by mass or more and 100% by mass or less.

13. The photochromic composition according to claim 1, wherein the photochromic composition consists of the first photochromic compound, the second photochromic compound A, the second photochromic compound B, and the second photochromic compound C, a content of the first photochromic compound is 1% by mass or more and 99% by mass or less, a content of the second photochromic compound A is 1% by mass or more and 99% by mass or less, a content of the second photochromic compound B is 1% by mass or more and 99% by mass or less, and a content of the second photochromic compound C is 1% by mass or more and 99% by mass or less.

14. The photochromic composition according to claim 1, wherein the photochromic composition comprises the second photochromic compound A.

15. A curable composition comprising:
the photochromic composition according to claim 1; and
at least one selected from the group consisting of a radical polymerizable monomer, a cationic polymerizable monomer, a compound having a polymerization reactive group, and a (thio)urethane(urea) polymer.

16. A cured product of the curable composition according to claim 15.

17. An optical article comprising the cured product according to claim 16.

18. A lens comprising the photochromic composition according to claim 1.

19. Eyeglasses comprising the lens according to claim 18.
